# EUROPEAN PATENT APPLICATION

(11) **EP 2 990 029 A1**
(43) Date of publication of application: **02.03.2016**
(21) Application number: 14182755.0
(22) Date of filing: 29.08.2014
(51) Int. Cl.: A61K 9/00, A61K 9/20, A61K 31/7042, A61P 3/10

(54) **Pharmaceutical compositions comprising Canagliflozin**

(71) Applicant: SANDOZ AG, 4056 Basel (CH)
(72) Inventor: The designation of the inventor has not yet been filed
(74) Representative: Kluschanzoff, Harald

(57) **Abstract**

The present invention belongs to the field of pharmaceutical industry and relates to a dry pharmaceutical composition comprising Canagliflozin, as well as to a process for preparing the same. Such dry pharmaceutical composition is useful as a medicament, especially for the normalization of plasma glucose levels.

## Description

### Field of the invention

The present invention belongs to the field of pharmaceutical industry and relates to a process of obtaining pharmaceutical compositions comprising Canagliflozin, as well as to pharmaceutical compositions comprising Canagliflozin in the form of non-stoichiometric hydrates or in amorphous form. Such pharmaceutical compositions are useful as a medicament, especially for the normalization of plasma glucose levels.

### Description of the background art

Canagliflozin (chemical name: 1-(β-D-glucopyranosyl)-4-methyl-3-[5-(4-fluorophenyl)-2-thienylmethyl]benzene) belongs to a novel therapeutic class of sodium-glucose co-transporter 2 inhibitors. A marketed pharmaceutical composition of Canagliflozin (INVOKANA^{®}) serves as an adjunct to diet and exercise to improve glycemic control in adults with type-2 diabetes mellitus. 1-(β-D-glucopyranosyl)-4-methyl-3-[5-(4-fluorophenyl)-2-thienylmethyl]benzene is represented by the following general formula (I):

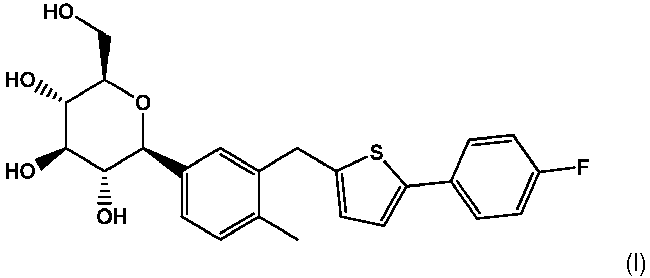

WO 2005/012326 A1 discloses the compound of formula (I) *per se.* A procedure for the preparation of the compound of formula (I) is disclosed generically in example 1 of the same application. However, the application is silent about the solid state form obtained.

WO 2008/069327 A1 discloses a crystalline hemihydrate of the compound of formula (I) and a process for the preparation thereof. This document further mentions that amorphous Canagliflozin suffers from stability and handling issues such as poor filterability.

WO 2009/035969 A1 discloses a crystalline form of the compound of formula (I). An overlay of X-ray powder diffraction (XRPD) of the crystalline form of the compound of formula (I) provided in figure 1 of WO 2009/035969 A1 and the crystalline form of the compound of formula (I) provided in figure 1 of WO 2008/069327 A1 shows good agreement, confirming the presence of the same solid state form, namely the crystalline hemihydrate. In addition a process for preparing the crystalline hemihydrate of the compound of formula (I) is disclosed.

WO 2012/154812 A1 discloses co-crystals of the compound of formula (I) with L-proline and citric acid and methods for their preparation.

WO 2013/064909 A2 discloses amorphous form of the compound of formula (I) as well as co-crystals with L-proline, D-proline and L-phenylalanine respectively. Processes for the preparation of these solid forms are also disclosed. However, no specific formulations are disclosed in this application.

Canagliflozin exists in various solid forms with different pharmacological and toxicological behaviors, and with variable bioavailabilties. These solid forms can be susceptible to transformation from one form to another, even at ambient conditions. Therefore, it is essential to prevent any modification of the solid forms of Canagliflozin during the shelf-life of the finished pharmaceutical composition. There is thus a need, and hence it is an object of the present invention, to provide pharmaceutical formulations or compositions of Canagliflozin, in which the polymorphic integrity of the drug is maintained during the entire shelf-life of the drug, so that the end user consistently gets the same desired effect upon repeated administration.

Also, it is required from the drug regulatory agencies to have the manufacturing methods of the drugs standardized and controlled in such a way that these give homogeneous results in terms of polymorphism. The modification in the amounts of these different solid forms in the finished pharmaceutical composition is highly critical as any variation in their amount during the shelf-life of the composition can directly affect the bioavailability of Canagliflozin in patients. It is an objection of the present invention to provide a process by which formulations or compositions of such Canagliflozin can efficiently be prepared. Furthermore, it is in general desirable that a pharmaceutical formulation or composition comprising an active pharmaceutical ingredient (API) has a sufficient chemical and physical stability to avoid formation of impurities and transformation to other solid forms. There is thus a need, and hence it is an object of the present invention, to provide pharmaceutical formulations or compositions of Canagliflozin, on the basis of which an improved stability becomes feasible. WO 2011/142478 discloses a tablet which comprises a high content of Canagliflozin preferably in the form of hemihydrate and a relatively small amount of additives to improve compactability of granules for tableting and tablet characteristics such as hardness, friability and disintegration, according to which a wet granulation is preferred for production of the tablets.

WO 2011/143296 discloses a formulation of Canagliflozin preferably in the form of hemihydrate and excipients including a diluent, a disintegrant, a binder and a lubricant. For the process of manufacturing the formulation a wet granulation technique is preferred.

None of the above prior art documents discloses information about stability of prepared compositions. The present inventors have now developed pharmaceutical compositions of Canagliflozin which shows good polymorphic stability, and which provide uniform therapeutic effect when administered to the patients.

Additionally, Canagliflozin is a poorly soluble compound with incomplete bioavailability. Poor drug solubility however represents a bottleneck for dissolution, which in turn critically affects drug bioavailability. Owing to the afore-described constraints in dissolution and bioavailability, the currently marketed formulation of Canagliflozin (INVOKANA^{®}) contains Canagliflozin as a hemihydrate form along with a diluent, a disintegrant, a binder and a lubricant. In the process of manufacturing the marketed formulation wet granulation technique is used. Yet, Canagliflozin hemihydrate is reported to exhibit incomplete bioavailability (65%) (see e.g. item 12.3 of INVOKANA^{®} US label). Therefore there is an additional need to develop a Canagliflozin formulation with improved bioavailability. This could be achieved by a formulation with improved solubility and/or dissolution rate compared to the existing marketed product. There is thus a need, and hence it is an object of the present invention, to provide pharmaceutical formulations or compositions of Canagliflozin, on the basis of which a faster dissolution and thus an improved bioavailability becomes feasible.

These and further objects, which will become apparent from the following description of the present invention, can be made possible by the subject-matter of the independent claims. Some of the preferred embodiments of the present invention are defined by the subject matter of the dependent claims.

### Summary of the invention

The present invention provides, as set forth in the following items, various aspects, subject-matters and preferred embodiments, which respectively taken alone or in combination, contribute to solving the object of the present invention as well as further objects:
(1) A formulation comprising a non-stoichiometric hydrate of Canagliflozin and at least one of excipients, (i) which formulation was obtained under dry atmosphere conditions (relative humidity (RH) of ≤ 40%) during processing; and/or (ii) wherein the formulation is defined by a water activity of ≤ 0.4.
(2) A formulation comprising a non-stoichiometric hydrate of Canagliflozin and at least one of excipients, (i) which formulation was obtained under dry atmosphere conditions (relative humidity (RH) of ≤ 30%) during processing; and/or (ii) wherein the formulation is defined by a water activity of ≤ 0.3.
(3) A formulation comprising a non-stoichiometric hydrate of Canagliflozin and at least one of excipients, wherein the formulation is defined by a water activity of ≤ 0.4.
(4) A formulation comprising a non-stoichiometric hydrate of Canagliflozin and at least one of excipients, wherein the formulation is defined by a water activity of ≤ 0.3.
(5) A formulation comprising a non-stoichiometric hydrate of Canagliflozin and at least one of excipients, wherein the formulation is defined by a water activity of ≤ 0.25.
(6) A formulation comprising a non-stoichiometric hydrate of Canagliflozin and at least one of excipients, wherein the formulation is defined by a water activity of ≤ 0.2.
(7) The formulation of any one of preceding items, wherein more than about 95%, about 96%, about 97%, about 98%, or about 99% of the Canagliflozin is stably present as a non-stoichiometric hydrate.
(8) The formulation of any one of preceding items, wherein the non-stoichiometric hydrate of Canagliflozin is HxA, HxB or a mixture of HxA and HxB, preferably is HxA.
(9) The formulation of any one of preceding items, wherein HxA is the only detectable crystalline form of Canagliflozin.
(10) A formulation comprising an amorphous Canagliflozin and at least one of excipients, (i) which formulation was obtained under dry atmosphere conditions (relative humidity (RH) of ≤ 40%) during processing; and/or (ii) wherein the formulation is defined by a water activity of ≤ 0.4.
(11) A formulation comprising an amorphous Canagliflozin and at least one of excipients, (i) which formulation was obtained under dry atmosphere conditions (relative humidity (RH) of ≤ 30%) during processing; and/or (ii) wherein the formulation is defined by a water activity of ≤ 0.3.
(12) A formulation comprising an amorphous Canagliflozin and at least one of excipients, wherein the formulation is defined by a water activity of ≤ 0.4.
(13) A formulation comprising an amorphous Canagliflozin and at least one of excipients, wherein the formulation is defined by a water activity of ≤ 0.3.
(14) A formulation comprising an amorphous Canagliflozin and at least one of excipients, wherein the formulation is defined by a water activity of ≤ 0.25.
(15) A formulation comprising an amorphous Canagliflozin and at least one of excipients, wherein the formulation is defined by a water activity of ≤ 0.2.
(16) The formulation of any one of preceding items, (i) wherein the amorphous Canagliflozin is unmicronized; or (ii) the amorphous Canagliflozin has a particle size in a range of d_{0.9} 2 - 1000 µm, preferably 30 - 700 µm.
(17) The formulation according to any of the preceding items, wherein the water activity is determined at room temperature.
(18) The formulation according to any of the preceding items, wherein the water activity is determined with a reference humidity measuring instrument, preferably with the measuring instrument testo 950.
(19) The formulation of any one of preceding items, wherein the at least one of excipients are selected from the group consisting of fillers, disintegrants, solubilizers, binders, lubricants and retardants.
(20) The formulation of any one of preceding items, wherein the at least one of excipients are selected from:
   fillers selected from the group consisting of microcrystalline cellulose, silicified microcrystalline cellulose and lactose monohydrate;
   disintegrants selected from the group consisting of croscarmellose sodium, crospovidone and low-substituted hydroxypropyl cellulose (L-HPC);
   solubilizers selected from the group consisting of surfactants and solubilizing polymers, preferably respectively selected from the group consisting of sodium lauryl sulfate and hydroxypropylmethyl cellulose (HPMC); and
   lubricants selected from magnesium stearate..
(21) The formulation of any one of preceding items, comprising as excipients sodium lauryl sulfate or a mixture of sodium lauryl sulfate and HPMC.
(22) The formulation of any one of preceding items, comprising as excipients:
   microcrystalline cellulose and/or silicified microcrystalline cellulose;
   croscarmellose sodium and/or crospovidone;
   sodium lauryl sulfate or a mixture of sodium lauryl sulfate and HPMC; and
   magnesium stearate.
(23) The formulation of item (21) or (22), wherein the concentration of sodium lauryl sulfate or each of sodium lauryl sulfate and HPMC in the mixture is more than 0.1% (w/v), preferably 0.5 to 2.0% (w/v), more preferably 0.8 to 1.2% (w/v), most preferably 1.0% (w/v).
(24) The formulation of any one of preceding items, wherein a water soluble lactose is absent.
(25) The formulation of any one of preceding items, which is a granulate.
(26) A pharmaceutical composition, comprising a formulation of any one of preceding items.
(27) The pharmaceutical composition of item (26), obtained by a dry manufacturing process, preferably wherein the dry manufacturing process is a direct compression, a dry granulation such as a roller compaction and a slugging, and a moisture activated dry granulation, more preferably is a direct compression or a dry granulation.
(28) The pharmaceutical composition of item (26) or (27), which is formulated in a single dosage form, preferably a tablet.
(29) The pharmaceutical composition of any one of items (26) to (28), which includes a coating, preferably a film coating.
(30) The pharmaceutical composition of item (29), wherein the coating represents 1.5 to 6% (w/w) of the formulation.
(31) The pharmaceutical composition of any one of items (26) to (30), which is incorporated into a moisture protective packaging.
(32) The pharmaceutical composition of item (31), wherein the moisture protective packaging is selected from the group consisting of aluminium blister, aclar blister, glass bottle containing desiccant and HDPE bottle containing desiccant.
(33) A process for manufacturing a formulation comprising Canagliflozin, (i) including dry atmosphere conditions (relative humidity (RH) of ≤ 40%) during processing; and/or (ii) wherein the resulting formulation is defined by a water activity of ≤ 0.4.
(34) The process of item (33), wherein (i) the dry atmosphere conditions during processing is RH of ≤ 30%; and/or (ii) the resulting formulation is defined by a water activity of ≤ 0.3, preferably of ≤ 0.2.
(35) The process of any one of preceding items, wherein Canagliflozin is in the form of a non-stoichiometric hydrate.
(36) The process of item (35), wherein the non-stoichiometric hydrate of Canagliflozin is HxA, HxB or a mixture of HxA and HxB, preferably is HxA.
(37) The process of any of items (35) or (36), wherein HxA is the only detectable crystalline form of Canagliflozin.
(38) The process of any of items (33) or (34), wherein Canagliflozin is in an amorphous form.
(39) The process of item (38), wherein the amorphous form is the only detectable form of Canagliflozin.
(40) The process of any one of preceding items, wherein the water activity is determined at room temperature.
(41) The process of any one of preceding items, wherein the water activity is determined with a reference humidity measuring instrument, preferably with the measuring instrument testo 950.
(42) The process of item (33) or (34), wherein said at least one of excipients includes at least one type of excipient whose water activity was reduced, preferably the at least one of excipients including the filler.
(43) The process of item (42), wherein reducing water activity involves (i) subjecting the excipients under dry humidity condition (RH of ≤ 15%) for 12 to 36 hours, and/or (ii) drying the excipients in a vacuum chamber at least for 4 hours, respectively at ambient temperature.
(44) A process for manufacturing a pharmaceutical composition comprising a non-stoichiometric hydrate of Canagliflozin or an amorphous form of Canagliflozin, which involves a dry manufacturing process.
(45) The process of item (44), wherein the dry manufacturing process is a direct compression, a dry granulation such as a roller compaction and a slugging, and a moisture activated dry granulation, preferably is a direct compression or a dry granulation.
(46) The process of item (44) or (45), comprising incorporation the pharmaceutical composition into a moisture protective packaging.
(47) The process of item (46), wherein the moisture protective packaging is selected from the group consisting of aluminium blister, aclar blister, glass bottle containing desiccant and HDPE bottle containing desiccant.
(48) The process of any of items (44) to (47), wherein the non-stoichiometric hydrate of Canagliflozin is HxA, HxB or a mixture of HxA and HxB, preferably is HxA.
(49) A package comprising at least one single dosage form comprising a non-stoichiometric hydrate of Canagliflozin or an amorphous Canagliflozin, which single dosage form was obtained through dry manufacturing process; wherein said at least one single dosage form is packed in a package sealed against vapor and moisture permeation, and preferably further protected against light exposure.
(50) The package of item (49), which is designed as a moisture protective packaging selected from the group consisting of aluminium blister, aclar blister, glass bottle containing desiccant and HDPE bottle containing desiccant.
(51) The process of any of items (49) or (50), wherein the non-stoichiometric hydrate of Canagliflozin is HxA, HxB or a mixture of HxA and HxB, preferably is HxA
(52) A pharmaceutical composition of any one of preceding items or a package of any one of preceding items for use as a medicament.
(53) A pharmaceutical composition of any one of preceding items or a package of any one of preceding items for use in the treatment of diabetes mellitus, diabetic retinopathy, diabetic neuropathy, diabetic nephropathy, delayed wound healing, insulin resistance, hyperglycemia, hyperinsulinemia, elevated blood levels of fatty acids, elevated blood levels of glycerol, hyperlipidemia, obesity, hypertriglyceridemia, Syndrome X, diabetic complications, atherosclerosis and/or hypertension.
(54) A pharmaceutical combination comprising an effective amount of a formulation of any one of preceding items and metformin.
(55) A pharmaceutical composition comprising Canagliflozin, wherein more than about 95%, about 96%, about 97%, about 98%, or about 99% of the Canagliflozin is stably present as a non-stoichiometric hydrate or in an amorphous form.
(56) The pharmaceutical composition according to item (55), wherein the non-stoichiometric hydrate of Canagliflozin is HxA, HxB or a mixture of HxA and HxB, preferably is HxA.
(57) The pharmaceutical composition according to item (55) or (56), wherein HxA is the only detectable crystalline form of Canagliflozin.
(58) The pharmaceutical composition of any of items (55) to (57), wherein the equilibrium relative humidity of the composition is below about 40%, 35%, 30%, or 25%, preferably below about 30%.
(59) The pharmaceutical composition of any of items (55) to (58), wherein the equilibrium relative humidity of the composition is from about 10% to 40%, preferably from about 10% to 30%.
(60) The pharmaceutical composition of any of items (55) to (59), wherein the equilibrium relative humidity is measured at room temperature, preferably at a temperature of 25 ± 1 °C.
(61) A container comprising a pharmaceutical composition according to any of items (55) to (60) and a means to keep the equilibrium relative humidity of the composition at below about 40%, preferably about 30%.
(62) The container of item (61), wherein the container in combination with the means to keep the equilibrium relative humidity of the composition at below 40%, preferably about 30%, is capable of maintaining the equilibrium relative humidity of the composition at below 40%, preferably about 30%, for at least 6 months, preferably at least 12 months, even more preferably at least 24 months, and most preferably at least 36 months.
(63) A process for preparing a pharmaceutical composition according to any of items (55) to (60) comprising the steps of
   a) mixing the crystalline HxA form or the amorphous form of Canagliflozin with one or more pharmaceutically acceptable excipients at dry atmosphere conditions (relative humidity (RH) of ≤ 40%) during processing; and/or (ii) wherein the formulation comprising the non-stoichiometric hydrate of Canagliflozin or the amorphous form of is defined by a water activity of ≤ 0.4, determined at room temperature;
   b) optionally granulating the mixture obtained in step a) at dry atmosphere conditions (relative humidity (RH) of ≤ 40%); and
   c) further processing the mixture obtained in step a) or the granulate obtained in step b) at dry atmosphere conditions (relative humidity (RH) of ≤ 40%) to obtain a pharmaceutical composition according to any of items (55) to (60).
(64) The process of item (63) wherein the mixture or granulate is processed into an oral dosage form.
(65) The process of item (64) wherein the oral dosage form is a capsule or a tablet.
(66) A pharmaceutical composition of any one of items (55) to (60) or a container of any one of items (61) or (62) for use as a medicament.
(67) A pharmaceutical composition of any one of items (55) to (60) or a container of any one of items (61) or (62) for use in the treatment of diabetes mellitus, diabetic retinopathy, diabetic neuropathy, diabetic nephropathy, delayed wound healing, insulin resistance, hyperglycemia, hyperinsulinemia, elevated blood levels of fatty acids, elevated blood levels of glycerol, hyperlipidemia, obesity, hypertriglyceridemia, Syndrome X, diabetic complications, atherosclerosis and/or hypertension.
(68) A pharmaceutical combination comprising an effective amount of a pharmaceutical composition of any one of items (55) to (60) and metformin.

### Brief Description of the Figures

Figure 1: Aqueous solubilities of Canagliflozin forms HxA and HxB of the present invention and Canagliflozin hemihydrate of WO 2008/069327 A1 (=Hy0.5) at 25.0 ± 1.0 °C
Figure 2: A comparison of Canagliflozin dissolution from Invokana^{®} and Example 1
Figure 3: A comparison of Canagliflozin dissolution from Invokana^{®} and Example 2
Figure 4: A comparison of Canagliflozin dissolution from Invokana^{®} and Example 3
Figure 5: A comparison of Canagliflozin dissolution from Invokana^{®} and Example 4
Figure 6: The % of precipitated Canagliflozin from Mcllvaine buffer pH 6.8 containing excipient (concentration 1% (w/v)) relatively to the Mcllvaine buffer pH 6.8 containing Canagliflozin and no excipient
Figure 7: A comparison of Canagliflozin dissolution from Invokana^{®} and Example 5
Figure 8: A comparison of Canagliflozin dissolution from Invokana^{®} and Example 6
Figure 9: A comparison of Canagliflozin dissolution from Invokana^{®} and Example 7
Figure 10: A comparison of Canagliflozin dissolution from Invokana^{®} and Example 8
Figure 11: Dissolution profiles of formulations in FaSSGF and FaSSiF media (top). Plasma concentration profiles of Canagliflozin (bottom): a) simulated profile (GastroPlus™) of Invokana^{®} 100 mg product; b) predicted plasma profile of Example 3 formulation.
Figure 12: Stability of dry formulation of Canagliflozin form HxA at 50°C after 14 days
Figure 13: XRPD of amorphous Canagliflozin
Figure 14: Stability of binary mixtures of crystalline Canagliflozin hemihydrate and excipients at 50 °C after 14 days
Figure 15: Stability of binary mixtures of amorphous Canagliflozin and excipients at 50°C after 14 days
Figure 16: Stability of dry formulation of amorphous Canagliflozin at 50°C after 14 days

### Detailed description of the invention

The present invention is now described in more detail by preferred embodiments and examples, which are however presented for illustrative purpose only and shall not be understood as limiting the scope of the present invention in any way.

The present invention overcomes shortcomings of the prior art formulations of the marketed Canagliflozin hemihydrate (INVOKANA^{®}), by providing a dry formulation comprising a non-stoichiometric hydrate of Canagliflozin and at least one of excipients. From such a formulation the active pharmaceutical ingredient (API, Canagliflozin) may quickly dissolve or be released if desired, thereby making it possible to provide high bioavailability and effectiveness.

Another beneficial effect has been found that the amorphous Canagliflozin exhibits a high solubility, thereby making it possible to provide improved dissolution rate. Canagliflozin is a poorly soluble compound. Poor drug solubility however may cause constraints in dissolution and consequently bioavailability. Although the currently marketed formulation of Canagliflozin (INVOKANA^{®}) contains Canagliflozin as a hemihydrate form along with a diluent, a disintegrant, a binder and a lubricant, the marketed formulation is reported to exhibit incomplete bioavailability (65%).

Moreover, a pharmaceutical formulation or composition comprising polymorphs as the API should preferably have sufficient chemical and physical stability to avoid formation of impurities and transformation to other polymorphs. An additional beneficial effect in this respect has been found that the dry formulations according to the present invention have good chemical and physical stability, thereby ensuring that an initially used solid form of Canagliflozin can be maintained during processing and storage.

In a first aspect, the present invention relates to a formulation comprising a non-stoichiometric hydrate or an amorphous form of Canagliflozin and at least one of excipients, (i) which formulation was obtained under dry atmosphere conditions (relative humidity (RH) of ≤ 40%) during processing; and/or (ii) wherein the formulation is defined by a water activity of ≤ 0.4, optionally determined at room temperature. In a preferred embodiment, the present invention relates to a formulation comprising a non-stoichiometric hydrate or an amorphous form of Canagliflozin and at least one of excipients, wherein the formulation is defined by a water activity of ≤ 0.4, preferably ≤ 0.3, more preferably ≤ 0.25 and even more preferably ≤ 0.2. Preferably, the water activity is determined at room temperature.

The term "dry formulation" as used herein means a preparation wherein the API (non-stoichiometric hydrate of Canagliflozin or amorphous form of Canagliflozin) and further excipients for a pharmaceutical formulation or composition have been processed while avoiding typical wet conditions, but using dry atmosphere conditions. The "dry atmosphere condition" is typically made by using limited relative humidity (RH) during processing, for example to a range of ≤ 40%, preferably of ≤ 30%, more preferably of ≤ 25%, even more preferably of ≤ 20%. The "dry atmosphere condition" during processing may preferably be met when the resulting formulation is defined by a water activity of ≤ 0.4, preferably of ≤ 0.3, more preferably of ≤ 0.2, optionally determined at room temperature, most preferably that the entity of a pharmaceutical composition comprising the dry formulation has the specified water activity.

In the present invention, the "dry technological process" alone is not sufficient if the atmosphere is only under normal atmosphere conditions.

The term "room temperature" or "ambient temperature" as used herein is understood to mean temperatures of 15 to 25°C (see e.g. European Pharmacopoeia 7.7, 1.2 (2013)).

The term "non-stoichiometric hydrate" as used herein refers to a crystalline form containing water in a non-stoichiometric manner. The amount of water incorporated into the crystal structure depends on the relative humidity of the surrounding atmosphere.

The term "water activity" as used herein is defined by the expression a_{w} = p/p₀, where p is the vapor pressure of water in the substance, and p₀ is the vapor pressure of pure water at the same temperature. Water activity can be measured by suitable water activity meter devices, for example instrument Testo 650 using sensor 0628.0024, both available and sold by EminTech (Helsingborg, Sweden). If not otherwise stated, the water activity referred to in the present specification is determined at room temperature.

In the present invention the "water activity" is determined for at least the entity of the formulation which comprises the non-stoichiometric hydrate or amorphous form of Canagliflozin. In most of the cases, the "water activity" is determined for the formulation comprising the API. The term "entity" as used herein may be understood as a part containing the API in a pharmaceutical dosage unit. Thus, the entity of the formulation comprising the API can be further processed for instance to obtain a film coating, bi-layer tablets, and capsules filled with a dry granulation, and so on.

Any excipients used in pharmaceutical industry can be used in the present invention. In a preferred embodiment of the present invention, at least one of excipients includes at least one type of excipient whose water activity was reduced, preferably wherein all other excipients are either dry *per se* (commercially available as "pre-dried" excipients) or reduced in water activity, preferably by at least 30%. Commercially available "pre-dried" excipients include e.g. Avicel pH 112 and ProSolv. Preferably, at least the filler used in the formulation according to the present invention has been pre-dried prior to formulating.

Suitable excipients may include, without being limited to, fillers, disintegrants, solubilizers, lubricants, retardants, glidants, film coating agents, binders etc. Other excipients known in the field of pharmaceutical compositions may also be used.

In the present invention, the formulation may include, without being limited to, a granulate, preferably a dry granulate. The pharmaceutical composition on the other hand may include, without being limited to, a further processed granulate, e.g., granulate mixed with further excipients including inner granules and external granule phases, compressed, or otherwise processed.

The formulations or compositions described herein may contain fillers selected from the group consisting of different grades of starches, such as maize starch, potato starch, rice starch, wheat starch, pregelatinized starch, fully pregelatinized starch; cellulose derivatives, such as microcrystalline cellulose or silicified microcrystalline cellulose; sugar alcohols such as mannitol, erythritol, sorbitol, xylitol; monosaccharides like glucose; oligosaccharides like sucrose and lactose such as lactose monohydrate, lactose anhydrous, spray dried lactose or anhydrous lactose; and calcium salts, such as calcium hydrogenphosphate. Preferable fillers include microcrystalline cellulose, silicified microcrystalline cellulose, lactose monohydrate, and a mixture thereof. More preferably, fillers are microcrystalline cellulose and/or silicified microcrystalline cellulose.

The formulations or compositions described herein may also comprise disintegrants selected from the group consisting of croscarmellose sodium, crospovidone, low substituted hydroxypropyl cellulose (L-HPC), povidone, sodium starch glycolate, and other starch derivatives, as well as disintegration aids such as ion exchange resin such as Polacrilin Potassium and Magnesium Aluminometasilicate. Preferable disintegrants include croscarmellose sodium, crospovidone, L-HPC, and a mixture thereof. More preferably, disintegrants are croscarmellose sodium and/or crospovidone.

The formulations or compositions described herein may also comprise solubilizers selected from the group consisting of surfactants and solubilizing polymers, preferably respectively selected from the group consisting of sodium lauryl sulfate, hydroxypropylmethyl cellulose (HPMC), polyethylene glycol having molecular weight in the range of about 2000 to 10000, polysorbates, fatty acid esters, esters of glycerol and fatty acids, esters of polyethylene glycol and fatty acids and castor oil ethoxylate. Preferable solubilizers include sodium lauryl sulfate, HPMC and a mixture thereof; more preferably sodium lauryl sulfate, or a mixture of sodium lauryl sulfate and HPMC. In another preferred embodiment, the concentration of the solubilizers is more than 0.1 % (w/v), preferably is 0.5 to 2.0% (w/v), more preferably is 0.8 to 1.2% (w/v), most preferably is 1.0% (w/v). When a mixture of the solubilizers is used, the concentration of each solubilizer may be in the afore-mentioned preferred range.

The formulations or compositions described herein may also comprise excipients modifying a release rate and bioavailability of the Canagliflozin, if desired, e.g. selected from retardants selected from the group consisting of cellulose derivatives such as low viscosity HPC, HPMC; preferably the retardant is sodium carboxymethyl cellulose.

Lubricants, if used, may be selected from the group consisting of stearic acid, talc, glyceryl behenate, sodium stearyl fumarate and magnesium stearate; particularly preferably the lubricant is magnesium stearate.

The formulations or compositions described herein may also comprise glidants, such as talc, silicon dioxide, magnesium silicate, sodium stearate or a mixture thereof; film coating agents, such as polyvinyl alcohol, HPMC, HPC or other polymers used in pharmaceutical industry for film coating, or a mixture thereof; binders, such as a mono-, di- or polysaccharide such as sugars and starch, a sugar alcohol or another polyol, lactose, saccharose, sorbitol, mannitol, starch, cellulose derivatives or a mixture thereof. Preferred binders are selected from the group consisting of povidone, HPC and HPMC (low viscosity).

In another preferred aspect, a water soluble lactose is absent in the dry formulation comprising Canagliflozin. Such water soluble lactose includes for instance lactose anhydrous.

In one aspect of the present invention, the formulation comprises Canagliflozin in the form of a non-stoichiometric hydrate. In a preferred aspect of the present invention, the non-stoichiometric hydrate of Canagliflozin is HxA, HxB or a mixture of HxA and HxB, preferably is HxA.

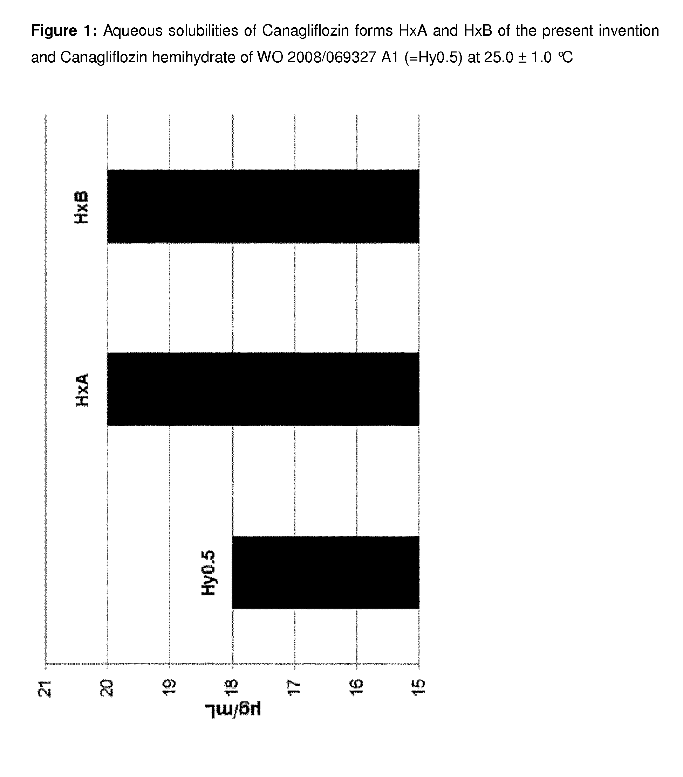

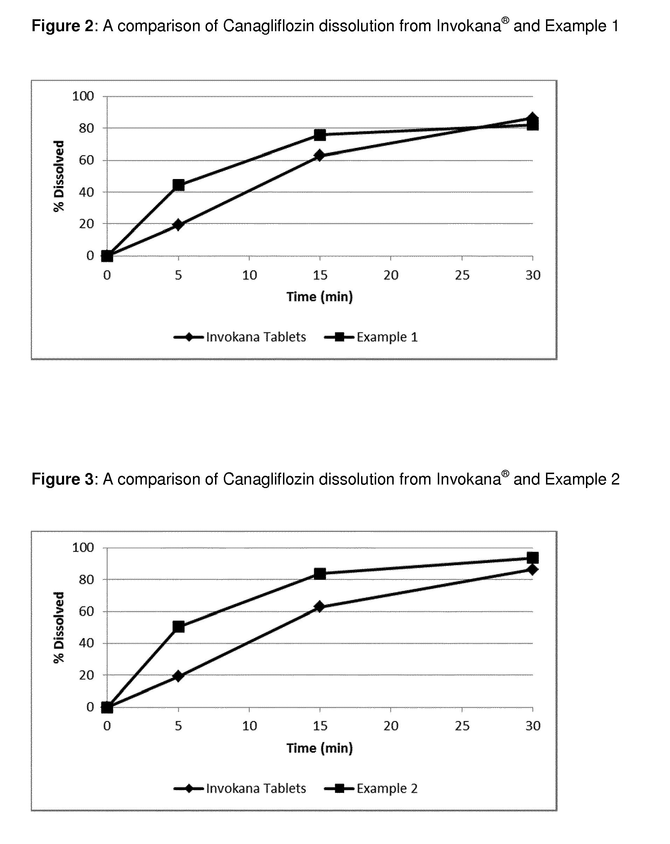

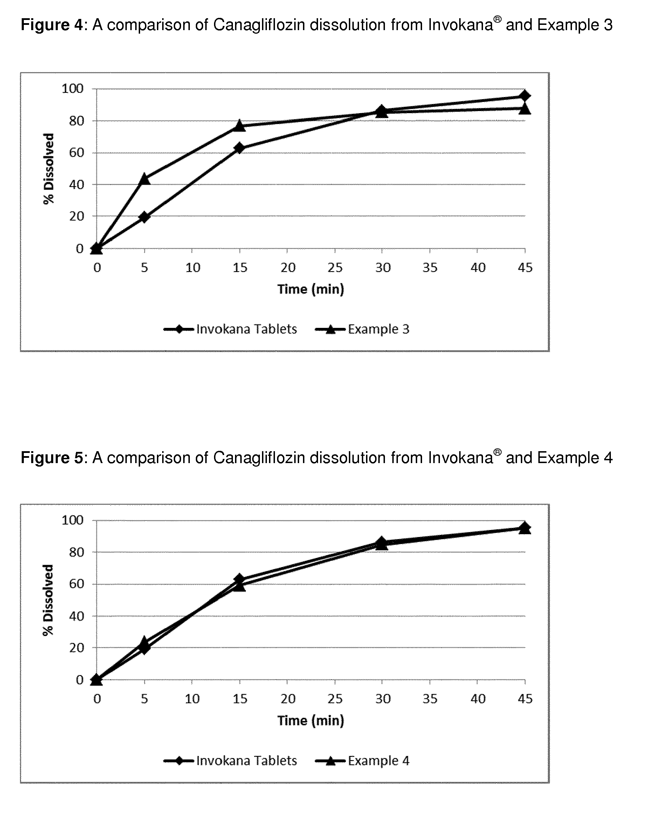

The crystalline non-stoichiometric hydrate forms "HxA" and "HxB" as used herein are disclosed in application no. PCT/EP2014/059281 (which is incorporated herein in its entirety). Referring to Figure 1 of the co-pending application, the form HxA is characterized by XRPD comprising characteristic peaks at 2-theta angles of 5.4 ± 0.2°, 6.7 ± 0.2°, 13.2 ± 0.2 °, 16.1 ± 0.2 °, 19.6 ± 0.2° and 24.1 ± 0.2 °. The form HxA can be characterized by a melting point having a peak maximum at about 86-87°C determined by DSC at a heating rate of 5°C/min (Figure 2 of the co-pending application). Further referring to Figure 3 of the co-pending application, the form HxB is characterized by XRPD comprising characteristic peaks at 2-theta angles of 6.6 ± 0.2 °, 7.3 ± 0.2 °, 12.2 ± 0.2 °, 15.4 ± 0.2 °, 19.9 ± 0.2° and 23.9 ± 0.2°. The form HxB can be characterized by a melting point having a peak maximum at about 82 °C determined by DSC at a heating rate of 5°C/min (Figure 4 of the co-pending application). The crystalline non-stoichiometric hydrate of Canagliflozin can be obtained by carrying out the steps of: (a) forming a suspension of amorphous Canagliflozin in water; (b) subjecting the obtained suspension to a particle size reduction process; and (c) isolating and subjecting the obtained crystals to an appropriate atmosphere condition.

In the present invention, the formulation may be, without being limited to, a granulate, preferably a dry granulate. The pharmaceutical composition on the other hand may be, without being limited to, a further processed granulate, e.g., granulate mixed with further excipients including inner granules and external granule phases, compressed, or otherwise processed.

In one aspect of the present invention, the formulation comprises Canagliflozin in an amorphous form. The amorphous Canagliflozin is unmicronized or micronized, preferably unmicronized in the formulations or compositions according to the present invention. In another aspect, the amorphous Canagliflozin has a particle size in a range of d_{0.9} 2 - 1000 µm, preferably 30 - 700 µm in the formulations or compositions according to the present invention.

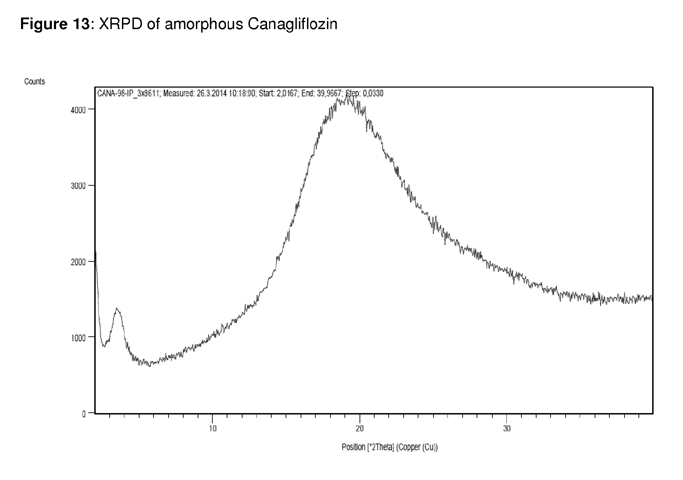

The amorphous form as used herein can be characterized by X-ray powder diffraction (XRPD) analysis, differential scanning calorimetry (DSC) traces, infrared (IR) spectroscopy, thermal gravimetric analysis (TGA), and so on. The amorphous form of Canagliflozin is for instance characterized by the XRPD pattern in accordance with Figure 13, which confirms that the material was amorphous as indicated by lack of any peaks. The amorphous form of Canagliflozin can be prepared for example in accordance with the procedures described in WO 2005/012326 A1.

In a particularly preferred aspect, the present invention therefore relates to a pharmaceutical composition comprising Canagliflozin wherein more than 95% of the Canagliflozin present in said composition is stably present as crystalline form HxA of Canagliflozin, more preferably to pharmaceutical compositions wherein crystalline form HxA of Canagliflozin is the only detectable crystalline from of Canagliflozin. "Stably present" as defined herein means that even after storage of the pharmaceutical composition for 180 days, and preferably even after storage for two years, the crystalline form HxA of Canagliflozin initially comprised in the pharmaceutical composition is still present as crystalline form HxA of Canagliflozin after storage for the indicated period. Such compositions can be produced by avoiding humid conditions, such as high relative humidity of the air, during the formulation steps. Furthermore, the above-identified humid conditions are to be avoided during storage in order to preserve the pharmaceutical composition of the invention.

In a preferred embodiment the pharmaceutical composition of the invention comprises the crystalline form HxA of Canagliflozin as the only detectable form of Canagliflozin. Analysis of the polymorphic state of Canagliflozin in a pharmaceutical composition can be performed by any suitable method known in the art, for example by XRPD.

It is further preferred that the pharmaceutical compositions of the invention exhibit an equilibrium relative humidity of below 40%, preferably of from 5% to 35%, more preferably of from 5% to 35%, most preferably from 5% to 30%, for at least 180 days, preferably for at least two years.

The equilibrium relative humidity of the pharmaceutical compositions as herein described is measured by determining the relative humidity in % in the air above a test sample, e.g. a pharmaceutical composition of the invention, after establishment of a humidity equilibrium in a closed system at a constant temperature according to the following method: the equipment used is the commercially available measuring chamber Rotronic AW-VC comprising a hygrometer of the type BT-RS1. The test sample, e.g. a pharmaceutical composition of the invention, is filled into a sampling dish which is placed into the measuring chamber which has been thermostated to room temperature (preferably of 25 +/- 1 °C), said chamber is subsequently closed and sealed. After establishment of an equilibrium of the relative humidity which state is typically shown by the disappearance of a trend indication, the value of the relative humidity in % is read from the hygrometer. Relative humidity is defined as the equilibrium relative humidity of the pharmaceutical compositions as measured as herein described. Filling of the chamber is to be performed in such a way as to provide complete filling of said chamber according to the instructions of the manufacturers. In case the test sample is a powder or granules for oral suspension, or a liquid suspension, said sample is directly placed into the above mentioned sampling dish. In case the test sample is a capsule, the appropriate number of capsules are opened and their contents is filled into the sampling dish. In case the test sample is a tablet, the appropriate number of tablets is crushed by using a mortar, and filled into the sampling dish. In cases where the equilibrium humidity is expected to be below 20%, the above described preparation of the test samples before measurement and the measurement itself as herein described is to be performed in a glove box being equipped with a hygrometer wherein a relative humidity of about 5% is to be established by e.g. flushing with dried air or nitrogen. The above described method for measurement of the equilibrium relative humidity of the pharmaceutical compositions of the invention is herein also called ERH method.

In a further aspect, the dry formulation comprising the non-stoichiometric hydrate of Canagliflozin or the amorphous Canagliflozin and at least one of excipients in accordance with the present invention can be further processed by using a optionally further dry manufacturing process to obtain a pharmaceutical composition, which may be formulated in a single dosage form such as tablets etc.

In another aspect of the invention, the entity of the dry formulation comprising the API can be further processed for instance to obtain a film coating, bi-layer or multi-layer tablets, a dosage form where the API-containing entity is separated to the outside or outer layers by a separation layer or a barrier layer, and capsules filled with a dry granulation, and so on. For instance when the entity is coated, basically typical coating techniques used in pharmaceutical industry can be used in the present invention. In a preferred embodiment of the present invention, the coating is a film coating which represents 1.5 to 6% (w/w) of the entity comprising the API. In a preferred embodiment, process conditions such as product temperature, spray rate, inlet air humidity etc. may be optimized when coatings containing water are used. As defined above, however, the "water activity" is determined for at least the entity of the formulation which comprises the API. Therefore, provided that the dry formulation, prior to coating, was obtained under dry atmosphere conditions, and/or the water activity of at least the entity of the formulation comprising the API is sufficiently low, a dry pharmaceutical composition according to the present invention can be obtained despite using coatings containing water.

Within the meaning of the present invention, the term "dry manufacturing process" indicates any manufacturing process for preparing a pharmaceutical composition from which typical wet conditions are refrained. Examples of the dry manufacturing process may include a direct compression, a dry granulation such as a roller compaction and a slugging, and a moisture activated dry granulation, preferably are a direct compression or a dry granulation.

Moisture-activated dry granulation (MADG) was developed in response to the difficulties experienced with wet granulation, in terms of endpoint, drying and milling, and offers an efficient, cost-effective granulating process that does not require a drying step. Essentially, the MADG process comprises two major steps: agglomeration and moisture distribution. During agglomeration, the active pharmaceutical ingredient (API) is dry blended with a binder or combination of binders in a typical high shear granulator. A limited amount of moisture (8%) is then added to granulate the powder mixture, but not enough to over-wet it. The water droplets hydrate the dry binder(s) and create tacky nuclei or a tacky wet mass, and the dry powder particle adhere to the tacky nuclei/mass to create moist agglomerates. During the moisture distribution stage, an absorbent excipient is added to evenly distribute the moisture and 'dry' the granulation. At this stage, it is still possible to add in any remaining drug or, if the formulation requires it, a lubricant or disintegrant. Finally, the contents can be lubricated, sieved/sized and blended.

In another preferred aspect, the pharmaceutical composition is incorporated into a moisture protective packaging, such as aluminium blisters, aclar blisters, glass bottles containing desiccant or HDPE bottles containing desiccant.

Surprisingly, it has been found that the dry pharmaceutical formulations or compositions according to the present invention may exhibit faster dissolution rates, as compared to the prior art formulation comprising Canagliflozin hemihydrate. For instance, as confirmed from Figures 2 to 5, the pharmaceutical formulations or compositions comprising a non-stoichiometric hydrate of Canagliflozin exhibit a dissolution profile by releasing Canagliflozin at a rate of at least 20% within 5 minutes, at least 40% within 10 minutes, at least 60% within 15 minutes, and at least 82% within 30 minutes, respectively relative to the original amount of Canagliflozin in the composition. Especially, initially high dissolution rates may contribute to improvement of bioavailability and effectiveness.

Surprisingly, it has also been found that the dry formulation or composition comprising the non-stoichiometric hydrate of Canagliflozin according to the present invention is chemically and physically more stable, compared to a situation where the dry atmosphere condition and/or the water activity is not properly observed. As a consequence, an initially used non-stoichiometric hydrate of Canagliflozin can be maintained according to the present invention during processing and storage.

Surprisingly, it has also been found that the dry formulation or composition comprising the amorphous Canagliflozin according to the present invention is chemically and physically more stable, compared to a situation where the dry atmosphere condition and/or the water activity is not properly observed. As a consequence, an initially used amorphous Canagliflozin can be maintained according to the present invention during processing and storage. Further surprisingly, it has been found that the amorphous Canagliflozin exhibits a proper dissolution profile independently from the particle size (micronization) against all expectations.

In the present invention, the dissolution testing may be performed according to any conventional methods, for example in an aqueous media buffered to an appropriate pH range without addition of a surfactant at an appropriate temperature (about 37±1 °C).

In a preferred embodiment of the invention, dissolution rate and/or stably dissolved status of the dry pharmaceutical composition according to present invention can be further adjusted or improved by using a substance capable of inhibiting precipitation of Canagliflozin. Preferably, the substance capable of inhibiting precipitation of Canagliflozin can be chosen from appropriate solubilizers as defined above. Further preferred, precipitation inhibition may concurrently be accomplished if appropriate solubilizers are chosen to be present in the formulation or composition, such as sodium lauryl sulfate, HPMC or a mixture of sodium lauryl sulfate and HPMC. Particularly beneficial precipitation inhibition has been found by a combination of Canagliflozin with the mixture of sodium lauryl sulphate and HPMC. In a further preferred aspect, the concentration of each substance capable of inhibiting precipitation may be more than 0.1 % (w/v), preferably 0.5 to 2.0% (w/v), more preferably 0.8 to 1.2% (w/v), most preferably 1.0% (w/v).

When desired, the dissolution profiles of the dry pharmaceutical composition according to present invention may be modified, for example by adding retardants such as Cellulose derivatives such as low viscosity HPC, HPMC; preferably sodium carboxymethyl cellulose.

Furthermore, the present invention provides a process for manufacturing a dry formulation comprising Canagliflozin and at least one of excipients, (i) which formulation was conducted under dry atmosphere conditions (relative humidity (RH) of ≤ 40%) during processing; and/or (ii) wherein at least the entity of the formulation which comprises the non-stoichiometric hydrate of Canagliflozin is defined by a water activity of ≤ 0.4, determined at room temperature.

Preferably, the present invention provides a process for manufacturing a dry formulation comprising Canagliflozin and at least one of excipients, (i) which formulation was conducted under dry atmosphere conditions (relative humidity (RH) of ≤ 30%) during processing; and/or (ii) wherein at least the entity of the formulation which comprises the non-stoichiometric hydrate of Canagliflozin is defined by a water activity of ≤ 0.3, determined at room temperature.

In accordance with another embodiment, the present invention provides a process for manufacturing a dry formulation comprising a non-stoichiometric hydrate of Canagliflozin or an amorphous Canagliflozin and at least one of excipients, (i) which formulation was conducted under dry atmosphere conditions (relative humidity (RH) of ≤ 40%) during processing; and/or (ii) wherein the formulation is defined by a water activity of ≤ 0.4, determined at room temperature. According to a further embodiment of the invention, said at least one of excipients includes at least one type of excipient whose water activity was reduced. Preferably, all other excipients are either dry *per se* (commercially available "pre-dried" excipients) or reduced in water activity by at least 30%. Preferably, reducing water activity by at least 30% involves (i) subjecting the excipients under dry humidity condition (RH of ≤ 15%) for 12 to 36 hours, and/or (ii) drying the excipients in a vacuum chamber at least for 4 hours, respectively at ambient temperature.

In a further aspect, the present invention relates to a package comprising at least one single dosage form comprising a non-stoichiometric hydrate of Canagliflozin or an amorphous Canagliflozin, which single dosage form was obtained through dry manufacturing process; wherein said at least one single dosage form is packed in a package sealed against vapor and moisture permeation, and preferably further protected against light exposure.

Thus, according to the present invention, a dry formulation or composition can be continuously ensured, not only during processing but throughout long storage times. This is particularly feasible when the single dosage forms, such as tablets, are packed or saved immediately after production within packages and especially blister packs, bottles or press- through package (PTP) that are essentially or totally impermeable towards water vapor and moisture.

Suitable packages are essentially or totally water vapor/moisture impermeable include, but are not limited to foil/foil packs such as aluminum/aluminum blister, HDPE (high density polyethylene bottles), sheets made of plastics having water vapor barrier properties improved such as coated poly(vinyl chloride) or polypropylene, laminated sheets of a polypropylene and a poly(vinylidene fluoride), and blister packs with a - typically thermoformed - blister base part known under the term "tropical blisters". Preferably, the blister packs according to the invention have cold-formed foil/foil blister design and further preferably have black base parts and/or covers, allowing up to 100% protection from moisture, oxygen and light. One element of the foil/foil blister pack comprises a lamination of plastic film (e.g. PVC or PE), adhesive, foil, adhesive, and an outer plastic film. The outer film, which can be PET or PVC, supports the thin aluminium layer and acts as the heat-seal layer. The aluminium layer usually consists of several very thin layers rather than a single thick one. The multiple layers help ensure that pinholes do not go all the way through the foil. They also increase the stretchability of the metal and facilitate the cold-stretching process. These multilayer webs are formed, filled, and sealed on a machine that performs these functions in sequence much as the thermoform-fill-seal machine does except that neither web is heated before the forming step. In the process of making the foil/foil blister pack, during the cold-forming process, the foil is shaped and molded around a plug to form a cavity.

The pharmaceutical formations or compositions according to the present invention are useful as a medicament, specifically for the treatment of diabetes mellitus, diabetic retinopathy, diabetic neuropathy, diabetic nephropathy, delayed wound healing, insulin resistance, hyperglycemia, hyperinsulinemia, elevated blood levels of fatty acids, elevated blood levels of glycerol, hyperlipidemia, obesity, hypertriglyceridemia, Syndrome X, diabetic complications, atherosclerosis and/or hypertension.

The pharmaceutical formations or compositions according to the present invention can be administered alone or in combination with other pharmaceutically active compounds such as further antidiabetic agents. In this case the formulation or composition of Canagliflozin and the additional antidiabetic agent can be administered either simultaneously or sequentially. For example the formulation or composition of Canagliflozin may be administered in combination with metformin, sulfonylurea, pioglitazone, insulin or mixtures thereof, most preferably in combination with metformin. Further, the Canagliflozin formulation of the present invention and other pharmaceutically active compounds can be formulated in a same pharmaceutical composition or in a respectively different pharmaceutical composition.

As described in the afore-mentioned co-pending application, the non-stoichiometric hydrates of Canagliflozin molecule show favorable physicochemical properties. For example, they are chemically stable, have favorable crystal habits and therefore show good handling properties such as good isolation, drying, flow and compaction properties. Furthermore, the bioavailability of a compound intended to be administered orally is dependent on the compound's solubility as well as the compound's permeability, according to the Biopharmaceutical Classification System (BCS). Surprisingly, the crystalline non-stoichiometric hydrates HxA and HxB of Canagliflozin of the present invention both show a 10% increased solubility in water compared to the crystalline Canagliflozin hemihydrate of WO 2008/069327 A1. Hence crystalline forms HxA and HxB are preferred for the preparation of an orally administered medicament since, according to the BCS, the oral bioavailabilities of the crystalline non-stoichiometric hydrates of the present invention are higher than that for the crystalline hemihydrate of WO 2008/069327 A1, which are confirmed by subsequent examples.

Another additional advantage of the present invention is that the amorphous Canagliflozin shows better solubilities than the known crystalline form of Canagliflozin hemihydrate. Accordingly, the dry pharmaceutical formulations or compositions according to the present invention may exhibit higher bioavailability when administered orally according to the Biopharmaceutical Classification System (BCS).

Another additional advantage of the present invention is that the dry formulation or composition comprising the non-stoichiometric hydrate of Canagliflozin or the amorphous Canagliflozin is chemically and physically stable. As a consequence, an initially used non-stoichiometric hydrate of Canagliflozin can be maintained during processing and storage.

### Examples

The following examples are merely illustrative of the present invention and they should not be considered as limiting the scope of the invention in any way, as these examples and other equivalents thereof will become apparent to those versed in the art in the light of the present disclosure, and the accompanying claims.

### EXAMPLE A - NON-STOICHIOMETRIC HYDRATES OF CANAGLIFLOZIN

### Preparation of non-stoichiometric hydrates of Canagliflozin

### Preparation Example 1 - Preparation of 1-(β-D-glucopyranosyl)-4-methyl-3-[5-(4-fluorophenyl)-2-thienylmethyl]benzene form HxA

A suspension of 5.0 g amorphous 1-(β-D-glucopyranosyl)-4-methyl-3-[5-(4-fluorophenyl)-2-thienylmethyl]benzene (for example prepared in accordance with the procedures described in WO 2005/012326 A1) in 500 mL water was stirred at room temperature for 18 hours using a magnetic stirrer. Thereafter the solid material was collected by filtration and dried at 40 °C under vacuum (≤ 30 mbar) for about 24 hours (< 30% relative humidity) to obtain 4.8 g (96% of theory) of 1-(β-D-glucopyranosyl)-4-methyl-3-[5-(4-fluorophenyl)-2-thienylmethyl]benzene form HxA.

### Preparation Example 2 - Preparation of 1-(β-D-glucopyranosyl)-4-methyl-3-[5-(4-fluorophenyl)-2-thienylmethyl]benzene form HxB

A suspension of 0.50 g amorphous 1-(β-D-glucopyranosyl)-4-methyl-3-[5-(4-fluorophenyl)-2-thienylmethyl]benzene (for example prepared in accordance with the procedures described in WO 2005/012326 A1) in 50 mL water was stirred at room temperature for 6 hours using a magnetic stirrer. Thereafter the solid material was collected by filtration, dried overnight at 40 °C under vacuum (≤ 30 mbar) and subsequently open stored overnight in a desiccator over a saturated ammonium chloride solution at 80 ± 5% RH to obtain 0.45 g (90% of theory) of 1-(β-D-glucopyranosyl)-4-methyl-3-[5-(4-fluorophenyl)-2-thienylmethyl]benzene form HxB.

### Aqueous solubility (reference example)

As described in Example 5 of the co-pending application PCT/EP2014/059281, the aqueous solubilities of forms HxA and HxB of the present invention and the hemihydrate of Canagliflozin described in WO 2008/069327 A1 were determined by the equilibrium solubility method. Saturated solutions of Canagliflozin, obtained by stirring 50 mg of each crystalline form in 50 mL water were prepared and stirred at 25 ± 1 °C. After 60 minutes of stirring, three samples were withdrawn, filtered through 0.45 µm syringe filters and the filtrates were directly analyzed by HPLC.
- Column:: C18 4.6x150 mm 5 µm
- Eluent A:: 0.1% formic acid-water solution
- Eluent B:: 0.1% formic acid-acetonitrile solution
- Injection vol.:: 100 µL
- Column Temp.:: 25 °C
- Wavelength:: 220 nm
- Pump flow:: 1.2 mL/min

**Gradient:**

| | | | | | | | |
|---|---|---|---|---|---|---|---|
| **Time [min]** | **0.0** | **20.0** | **22.0** | **22.5** | **25.0** | **25.3** | **30.0** |
| **[%] B** | **20** | **80** | **80** | **95** | **95** | **20** | **20** |

Using six concentration points of known Canagliflozin concentrations a calibration line was fitted by linear regression. Forms HxA and HxB of Canagliflozin were found to have a 10% increased aqueous solubility compared to the crystalline hemihydrate described in WO 2008/069327 A1. The results are summarized in table 1.

**Table 1: Equilibrium aqueous solubilities at 25 ± 1 °C**

| **Crystalline Form** | **Abs. Solubilities [µg/mL]** | **Rel. Solubilities [%]** |
|---|---|---|
| **hemihydrate of** WO 2008/069327 A1 **(=Hy0.5)** | **18** | **90** |
| **form HxA** | **20** | **100** |
| **form HxB** | **20** | **100** |

### Dissolution rate

To assess bioavailability of prepared examples, dissolution rate of API in FaSSIF (fasted state simulated intestinal fluid) with pH 6.5 was measured. This medium contains bile salts, which mimics gastrointestinal conditions. Thus, in-vitro dissolution testing in FaSSIF is applicable for prediction of bioavailability. Dissolution performances of prepared samples were compared to Invokana^{®} tablet. Apparatus 2 (paddle method); 75 rpm and 500 ml of dissolution media has been used.

### Examples 1 to 4

Dry mixtures of Canagliflozin HxA and excipients were prepared according to composition presented in Tables 2 and 3 below (prepared at dry humidity conditions, i.e. ambient temperature, RH < 15%). Excipients were exposed to RH < 15% for 24 hours before preparation of samples. Tablets of 200 mg were compressed and subjected to the dissolution test.

**Table 2**

| | **Example 1** | **Example 2** |
|---|---|---|
| Canagliflozin (mg) | 100 | 100 |
| Crospovidone (mg) | 10 | / |
| Croscarmellose sodium (mg) | / | 10 |
| L HPC LH11 (mg) | 10 | 10 |
| Mg stearate (mg) | 2 | 2 |
| Avicel PH102 (mg) | 39 | / |
| ProSolv SMCC 90 (mg) | / | 78 |
| Lactose anhydrous (mg) | 39 | / |
| Total (mg) | 200 | 200 |

**Table 3**

| | **Example 3** | **Example 4** |
|---|---|---|
| Canagliflozin (mg) | 100 | 100 |
| Croscarmellose sodium (mg) | 10 | 10 |
| L HPC LH11 (mg) | 10 | 10 |
| Mg stearate (mg) | 2 | 2 |
| ProSolv SMCC 90 (mg) | 73 | 73 |
| Sodium Lauryl Sulfate (mg) | 5 | / |
| Poloxamer (mg) | / | 5 |
| Total (mg) | 200 | 200 |
| PSD of Canagliflozin (µm) | micronized | unmicronized |

As can be seen from Figures 2 to 5, the pharmaceutical formulation according to the present invention exhibited faster dissolution as compared to Invokana^{®}. More surprisingly, the initial dissolution rate was very fast, which contributes to improved bioavailability.

### Precipitation inhibition screening

In vitro high-throughput precipitation inhibition screening with nephelometer was performed for different excipients in Mcllvaine buffer pH 6.8 at 1 %, 0.1 %, and 0.01 % (w/v) according to the procedure published in Petru 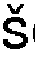 evska et al (Petru 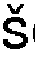 evska et al. Evaluation of light scattering and turbidity microtiter plate-based methods for detection of the excipient-mediated drug precipitation inhibition. Eur J Pharm Biopharm, 2013). The ratio between turbidity signal for each tested concentration of selected excipient with Canagliflozin and plain buffer containing only Canagliflozin were determined. Excipients added at 0.1 and 0.01 % (w/v) had negligible effects on inhibiting precipitation of Canagliflozin.

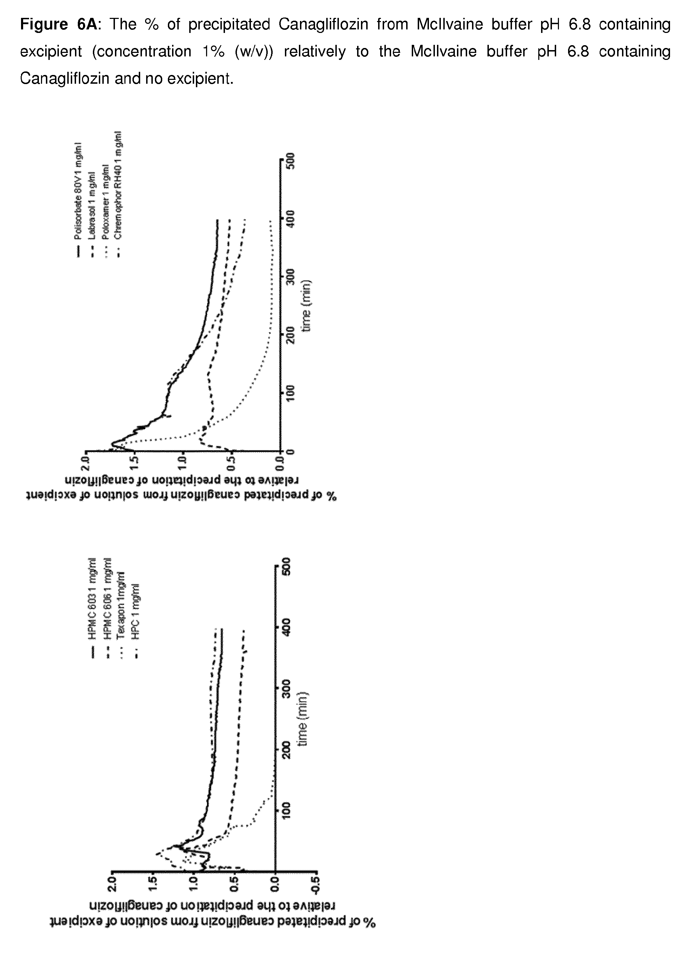

At the highest concentration tested, HPMC 603, HPMC 606, Texapon and Labrasol sustained Canagliflozin supersaturation for a short period of time (i.e. 30 min for HPMC 603, 10 min for HPMC 603, Texapon, and Labrasol), as shown in Figure 6A. The rate of canagliflozin dissolution was the fastest in the presence of HPMC 606, Texapon, and Poloxamer. Because HPMC 606 and Texapon could sustain initial canagliflozin supersaturation and provided the fastest canagliflozin dissolution, their combination (each excipient was present in 1 mg/ml concentration) was subjected to further turbidity measurements.

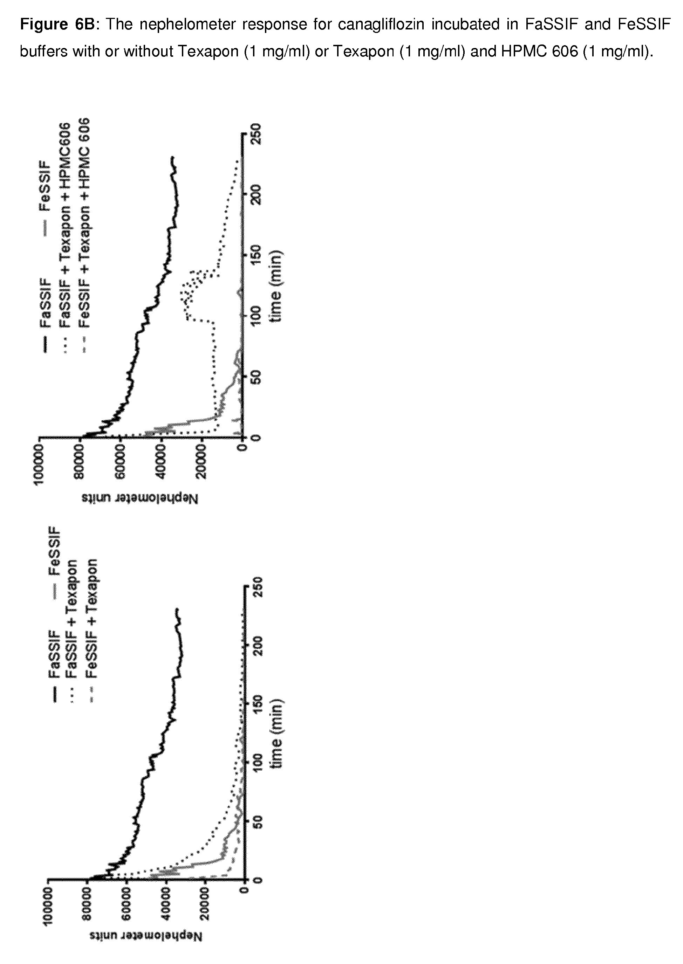

Inhibition of precipitation and supersaturation effects were also evaluated in more biorelevant dissolution media, FaSSIF and FeSSIF, prepared according to Dressman et al. (Dressman et al. Dissolution testing as a prognostic tool for oral drug absorption: immediate release dosage forms. Pharm Res, 1998)). Excipients were used in 1 mg/ml concentration. Results are shown as means ± standard deviations. All tests were performed in duplicate. As a result, the addition of Texapon or combination of Texapon and HPMC 606 significantly increased the rate of Canagliflozin dissolution (see equilibrium solubilities, determined at 37°C after 24h incubation of Table 4 below and Figure 6B).

**Table 4**

| Solubility media | Equilibrium solubility in Mcllvaine buffer pH 6.5 [mg/ml] | Equilibrium solubility in FaSSIF pH 6.5 [mg/ml] | Equilibrium solubility in FeSSIF pH 5.5 [mg/ml] |
|---|---|---|---|
| buffer | 0.021 ± 0.001 | 5.31 ±0.97 | 21.03 ± 1.03 |
| Texapon | 2.847 ± 0.332 | 7.98 | 24.98 ± 1.34 |
| HPMC 606 | 0.062 ± 0.007 | / | / |
| Texapon + HPMC 603 | 2.575 ± 0.036 | 8.48 ± 0.18 | 27.81 ± 2.35 |

### Modification of dissolution rate

Dry mixtures of Canagliflozin and excipients were prepared according to composition presented in Table 5 below (prepared at dry humidity conditions, i.e. ambient temperature, RH < 15%). Excipients were exposed to RH < 15% for 24 hours before preparation of samples. Tablets of 200 mg were compressed and subjected to the dissolution test.

**Table 5**

| | **Example 5** | **Example 6** | **Example 7** | **Example 8** |
|---|---|---|---|---|
| Canagliflozin (mg) | 100 | 100 | 50+50 | 50+50 |
| Croscarmellose sodium (mg) | 10 | 10 | 10 | 10 |
| L HPC LH11 (mg) | 10 | 10 | 10 | 10 |
| Mg stearate (mg) | 2 | 2 | 2 | 2 |
| ProSolv SMCC 90 (mg) | 56 | 56 | 67 | 67 |
| Sodium Lauryl Sulfate (mg) | 5 | / | 2.5 | 2.5 |
| Poloxamer (mg) | / | 5 | / | / |
| Blanose LF (mg) | 17 | 17 | 8.5 | 8.5 |
| Total (mg) | 200 | 200 | 200 | 200 |
| PSD of Canagliflozin (µm) | micronized | unmicronized | half micronized + half unmicronized | half micronized + half unmicronized |

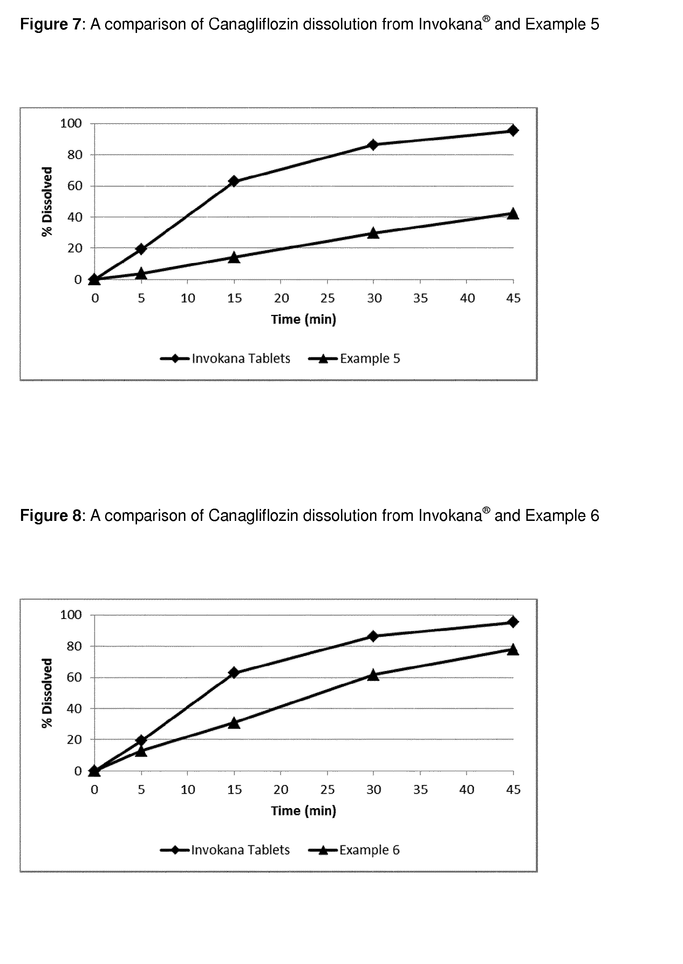

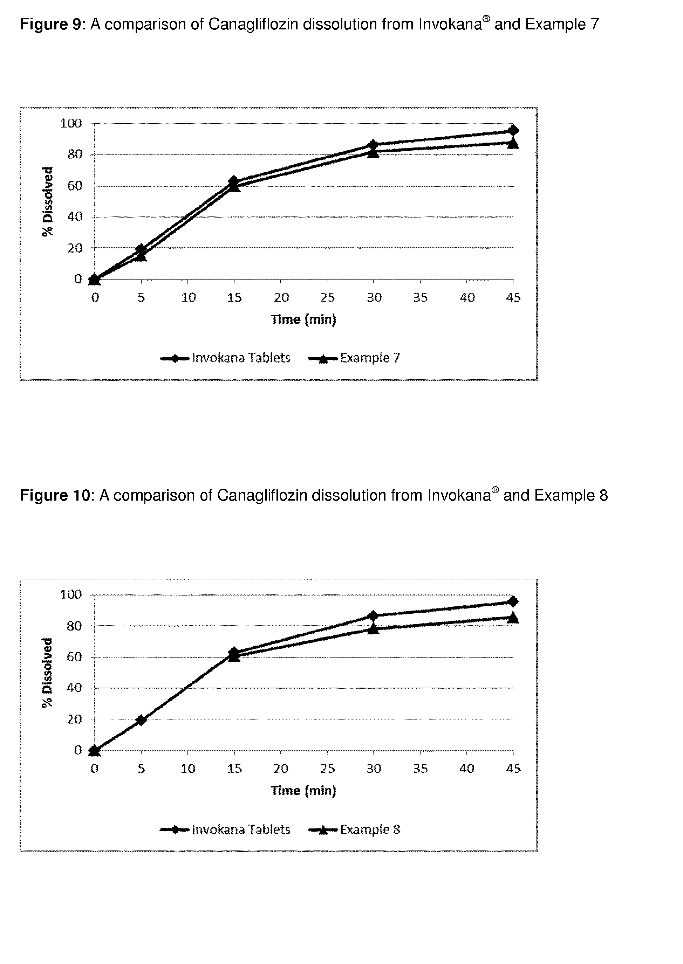

As can be seen from Figures 7 to 10, retardants have influence on dissolution profile. Thus, by the addition of retardants the dissolution rate of the formulations or composition according to the present invention could be adjusted as desired. It could be further surprisingly observed that particle size distribution did not necessarily influence the dissolution rate (not shown).

### Bioavailability

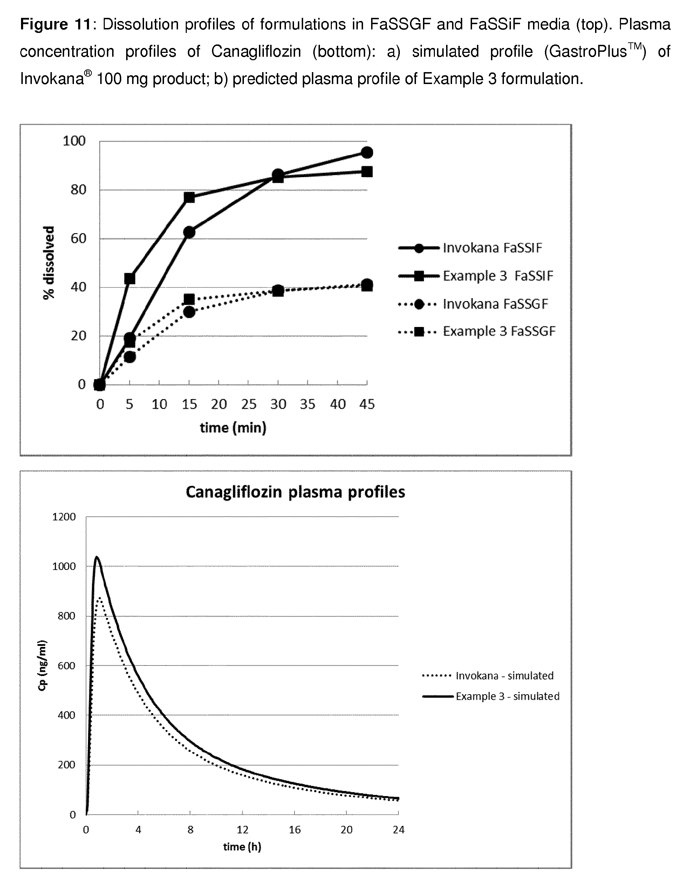

The beneficial effect of faster dissolution on the improved bioavailability was demonstrated by physiologically based pharmacokinetic computer modelling. A computer model was developed to predict in vivo performance of Canagliflozin formulations by the GastroPlusTM software (Simulations Plus Inc.). The dissolution profiles of formulations in FaSSGF and FaSSiF media were used as inputs for the model. The results are shown in Figure. 11.

The predicted plasma profile of the formulation according to Example 3 was significantly higher compared to the Invokana^{®} profile. It is estimated based on simulations that the formulation according to Example 3 improves the bioavailability of Canagliflozin for about 20% in terms of Cmax and about 15% in terms of AUC, relative to the Invokana^{®} product.

### Stability test

### Chemical stability

Canagliflozin degradation products were followed by high performance liquid chromatography using the following chromatographic method, as follows. Formulations were dissolved in a mixture of 60 w/w% acetonitrile in buffer pH=5 (0.77 g of CH₃OONH₄ in 1 L of water, pH adjusted to pH=5 with acetic acid) to achieve a concentration of about 0.2 mg/ml of Canagliflozin. The sample solution was injected into an UHPLC system with a BEH C18 column (1.7 micrometer particles) using binary gradient elution. Mobile phase A consisted of mixture of 70 w/w% buffer with pH=5 in acetonitrile and mobile phase B consisted of acetonitrile. Gradient elution was performed according to the following program: mobile phase A (%) / time (min): 100%/0min; 100%/5min; 14%/93min; 40%/35min; 15%/40min; 15%/45min. The detector was set to a wavelength of 290 nm and impurities quantitated as area % with no response factors applied. Stability of formulations was monitored by exposing them to elevated temperature (50°C) in a sealed glass vial for 14 days. After storage, formulations were analyzed and the amounts of degradation products measured by UHPLC. The extent of degradation was determined by subtracting the total amount of degradation products of a stressed sample from the total amount of degradation of a stressed sample.

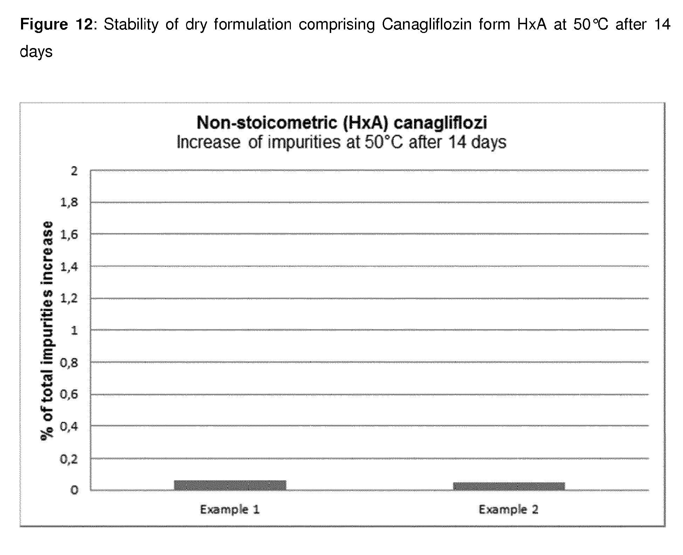

As shown in Figure 12, the dry formulations of Examples 1 and 2 were chemically stable as no increase of impurities were observed.

### Physical stability

Physical stability of polymorphic form of Canagliflozin was examined by XRD and dryness of the samples was measured by determination of water activity.

Diffraction patterns of samples were recorded using PANalytical X'Pert PRO MPD diffractometer with X'Celerator detector, CuKα radiation (tube operating at 45 kV and 40 mA) in the Bragg-Brentano (reflection) geometry. Data were recorded from 2 to 40 deg. 2theta in steps of 0.033 deg. 2theta and the measurement time of 50 seconds per step. Variable divergence and antiscatter slits were used to maintain 10 mm of sample length irradiated.

The dry formulations of Examples 1 and 2 having low water activity (below 0.2) were physically stable since transformation of the hydrate form was not observed as shown in Table 6.

**Table 6**

| Samples | Water activity of the tablets | XRD results on polymorphic form After storage at 50 °C for 14 days |
|---|---|---|
| Example 1 (HxA) | 0.16 | Non-stoichiometric (HxA) Canagliflozin |
| Example 2 (HxA) | 0.17 | Non-stoichiometric (HxA) Canagliflozin |

### Influence of water activity on physical stability of Canagliflozin

The influence of water activity on physical stability of Canagliflozin were further tested. Prior storage at stress stability conditions samples were exposed to different relative humidity (30, 40, 50 and 60 RH %). After storage at elevated temperature (50°C) for 14 days samples were tested for water activity and polymorphic form. It was established that Canagliflozin was physically stable when water activity was ≤ 0.3. Results confirmed that appropriate dryness of the samples is critical for product stability.

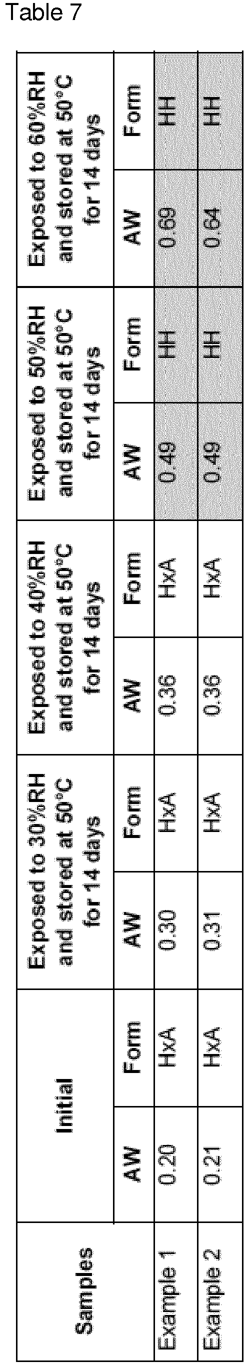

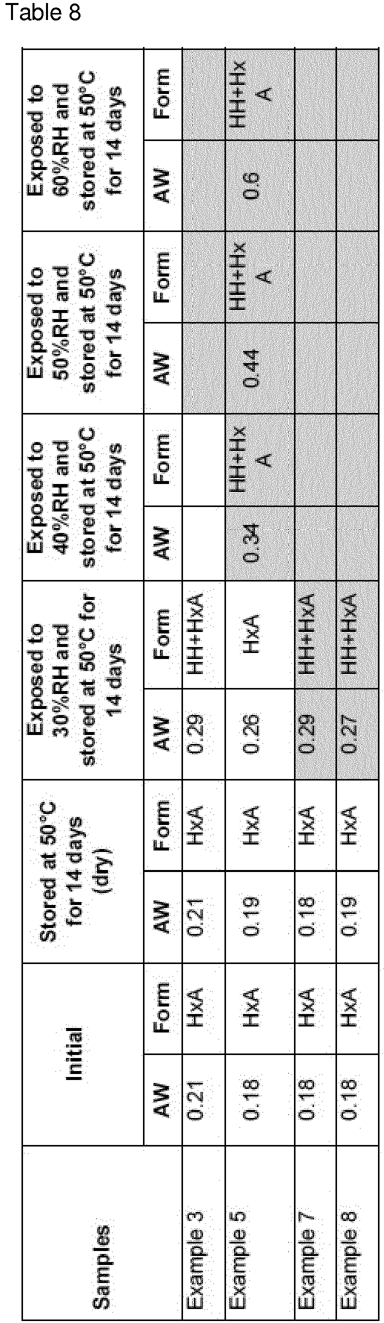

### EXAMPLE B - AMORPHOUS CANAGLIFLOZIN

### Preparation of amorphous Canagliflozin

85 g Canagliflozin (for example prepared in accordance with the procedures described in WO 2005/012326 A1) was dissolved in 250 mL of acetone at 40 °C. The solution was filtered thru Büchner filter funnel and solvent evaporated using reduced pressure and heating temperature of 30°C. Formed solid was additionally dried at 24°C for 3 days.

### Aqueous solubility

The solubilities of the amorphous Canagliflozin and the hemihydrate of Canagliflozin described in WO 2008/069327 A1 were determined at pH 1.2, 4.5 and 6.8 (37°C, shaking, HPLC determination). Additionally dynamic solubility in water was determined at 0.5h, 1 h and 24h time points (37°C, shaking, HPLC determination). The amorphous Canagliflozin was found to have increased solubility as well as dynamic solubility as compared to the crystalline Canagliflozin hemihydrate of WO 2008/069327 A1.

### Stability test

### Chemical stability

Canagliflozin degradation products were followed by high performance liquid chromatography using the following chromatographic method. Samples were dissolved in a mixture of 60 w/w% acetonitrile in buffer pH=5 (0.77 g of CH₃OONH₄ in 1 L of water, pH adjusted to pH=5 with acetic acid) to achieve a concentration of about 0.2 mg/ml of Canagliflozin. The sample solution was injected into an UHPLC system with a BEH C18 column (1.7 micrometer particles) using binary gradient elution. Mobile phase A consisted of mixture of 70 w/w% buffer with pH=5 in acetonitrile and mobile phase B consisted of acetonitrile. Gradient elution was performed according to the following program: mobile phase A (%) / time (min): 100%/0min; 100%/5min; 14%/93min; 40%/35min; 15%/40min; 15%/45min. The detector was set to a wavelength of 290 nm and impurities quantitated as area % with no response factors applied. Stability of samples was monitored by exposing them to elevated temperature (50°C) in a sealed glass vial for 14 days. After storage, samples were analysed and the amounts of degradation products measured by UHPLC. The extent of degradation was determined by subtracting the total amount of degradation products of a stressed sample from the total amount of degradation of a stressed sample.

### Chemical stability of amorphous Canagliflozin molecule in the binary mixture

To assess chemical stabilities of the amorphous Canagliflozin and the hemihydrate of Canagliflozin described in WO 2008/069327 A1, binary mixtures of each form of Canagliflozin and different excipients were prepared as follows: 1 g of Canagliflozin and 1 g of tested excipient were homogenized, and tablets of approximately 300 mg were compressed. The tested excipients are shown in Table 9.

**Table 9**

| **Sample** | **Name of excipient** | **Synonym** |
|---|---|---|
| **BM1** | Aerosil 200 | Colloidal Silicon Dioxide |
| **BM2** | Syloid AL-1 | Colloidal Silicon Dioxide |
| **BM3** | Primojel | Sodium Starch Glycolate |
| **BM4** | Ac-di-sol | Croscarmellose sodium |
| **BM5** | Polyplasodone XL | Crospovidone |
| **BM6** | Avicel PH 101 | Microcrystalline Cellulose |
| **BM7** | ProSolv smcc 90 | Silicified Microcrystalline Cellulose |
| **BM8** | Pharmacoat 603 | Hypromellose |
| **BM9** | Pharmacoat 606 | Hypromellose |
| **BM10** | HPC (Klucel EXF) | Hydroxypropyl Cellulose |
| **BM11** | Lactose spray dried | Lactose monohydrate |
| **BM12** | Lactose 70-100 MESH | Lactose monohydrate |
| **BM13** | PEG 6000 | Polyethylene Glycol 6000 |
| **BM14** | Texapon K12 | Sodium Lauryl Sulfate |
| **BM15** | Polivinilpirolidon K25 | Povidone |
| **BM16** | Kollicoat IR | Macrogol Poly(Vinyl Alcohol) Grafted Copolymer |
| **BM17** | Talc | Talc |
| **BM18** | Mannitol | Mannitol |
| **BM19** | Cremophor RH 40 | Polyoxyl 40 hydrogenated castor oil |
| **BM20** | Lutrol F68 | Poloxamer |
| **BM21** | Neosorb P 20/60 DC | Sorbitol |
| **BM22** | Polyvinyl alcohol 4-88 | Polyvinyl alcohol |

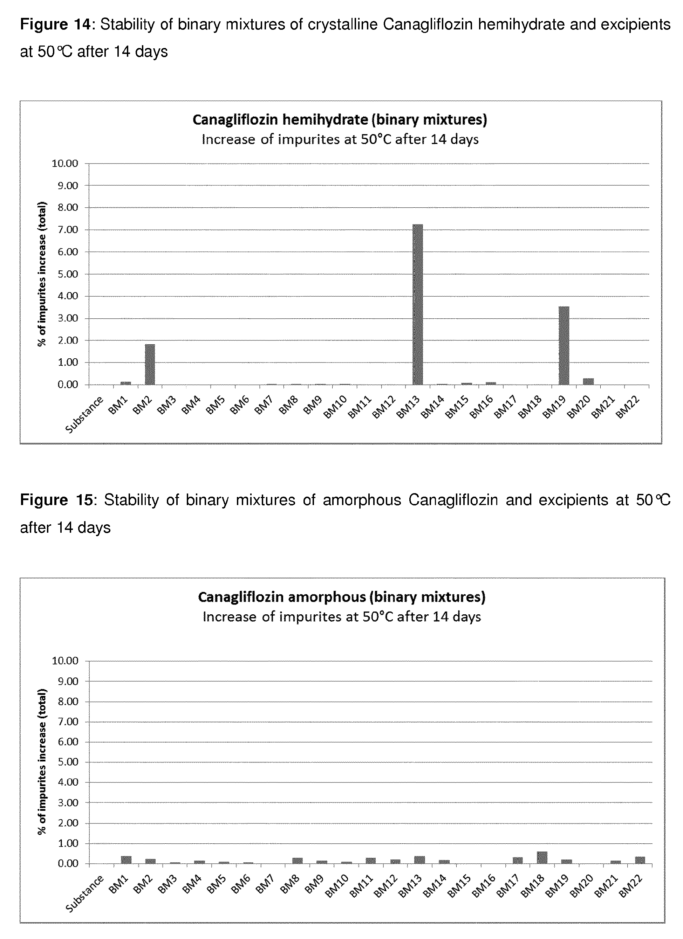

Results of degradation products of amorphous Canagliflozin and Canagliflozin hemihydrate are respectively shown in Figures 14 and 15. In mixtures with Canagliflozin hemihydrate, impurities were increased when containing excipients such as Syloid, PEG 6000 and Polyoxyl 40 hydrogenated castor oil (Figure 14). Surprisingly, in the mixtures prepared with amorphous Canagliflozin, no increase in impurities was observed (Figure 15). Accordingly, since the chemical stability of the amorphous Canagliflozin is less critical as compared to the crystalline Canagliflozin hemihydrate, more freedom of choice of excipients and hence options for improvement in other aspects are possible according to the present invention.

### Chemical stability of amorphous Canagliflozin molecule in the binary mixture

The Chemical stability of amorphous Canagliflozin contained in the dry formulation according to the present invention was assessed. 10 samples were prepared according to the composition as shown in Table 10.

**Table 10**

| | **Canagliflozin (mg)** | **Crospovidone (mg)** | **Croscarmellose sodium (mg)** | **L HPC LH11 (mg)** | **Mg stearate (mg)** | **Avicel PH102 (mg)** | **ProSdv SMCC 90 (mg)** | **Lactose anhydrous (mg)** | **Total (mg)** |
|---|---|---|---|---|---|---|---|---|---|
| **Example 1** | 100 | 10 | / | 10 | 2 | 78 | / | / | 200 |
| **Example 2** | 100 | 10 | / | 10 | 2 | 39 | / | 39 | 200 |
| **Example** 3 | 100 | / | 10 | 10 | 2 | 78 | / | / | 200 |
| **Example 4** | 100 | / | 10 | 10 | 2 | 39 | / | 39 | 200 |
| **Example 5** | 100 | 10 | / | 10 | 2 | | 78 | | 200 |
| **Example 6** | 100 | 10 | / | 10 | 2 | | 39 | 39 | 200 |
| **Example 7** | 100 | / | 10 | 10 | 2 | / | 78 | / | 200 |
| **Example 8** | 100 | / | 10 | 10 | 2 | / | 39 | 39 | 200 |
| **Example 9** | 100 | 10 | / | 10 | 2 | 58.5 | / | 19.5 | 200 |
| **Example 10** | 100 | 10 | / | 10 | 2 | 58.5 | / | 19.5 | 200 |

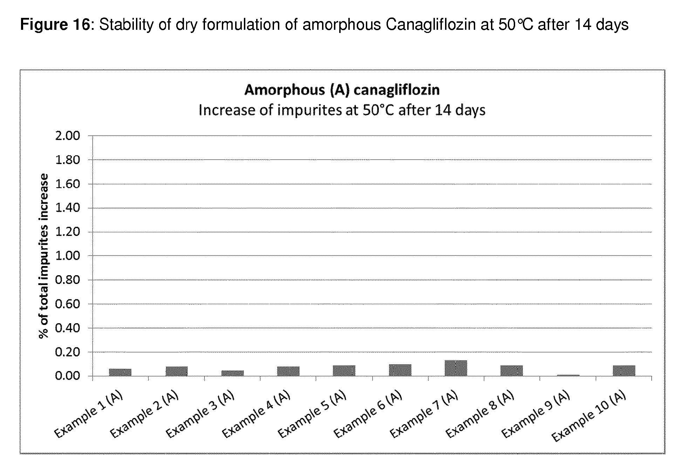

Samples according to Examples 1 to 10 were prepared as follows: dry mixture of amorphous Canagliflozin and excipients was prepared, and tablets of 200 mg were compressed. As shown in Figure 16, the dry formulations of Examples 1 to 10 were stable as no increase of impurities was observed.

### Physical stability

Physical stability of the dry formulation comprising amorphous Canagliflozin according to the present invention was examined by XRD (X'Pert PRO MPD, reflection technique, CuKα radiation, range 2 - 40 ° 2Θ, step 0,033° 2Θ, integr. time 50 s) and dryness of the samples was measured by determination of water activity.

The dry formulations of Examples 1 to 10 having low water activity (below 0.2) were physically stable since transformation of the hydrate form was not observed as shown in Table 11.

**Table 11**

| **Samples** | **Water activity** of the tablets | **XRD results on polymorphic form** After storage at 50 °C for 14 days |
|---|---|---|
| Example 1 | 0.16 | Amorphous Canagliflozin |
| Example 2 | 0.15 | Amorphous Canagliflozin |
| Example 3 | 0.14 | Amorphous Canagliflozin |
| Example 4 | 0.16 | Amorphous Canagliflozin |
| Example 5 | 0.15 | Amorphous Canagliflozin |
| Example 6 | 0.17 | Amorphous Canagliflozin |
| Example 7 | 0.18 | Amorphous Canagliflozin |
| Example 8 | 0.14 | Amorphous Canagliflozin |
| Example 9 | 0.15 | Amorphous Canagliflozin |
| Example 10 | 0.14 | Amorphous Canagliflozin |

### Influence of water activity of each excipient on stability

The influence of water activity of each excipient on chemical and physical stability of Canagliflozin was further tested.

**Table 12**

| **Samples** | **Disintegrant** | **Filler** | **Dried excipients** |
|---|---|---|---|
| Example 1 | Crospovidone | Avicel PH 102 | Normal* |
| Example 2 | Crospovidone | Avicel PH 102 | Dry# |
| Example 3 | Croscarmellose sodium | Avicel PH 102 | Dry |
| Example 4 | Croscarmellose sodium | Avicel PH 102 | Normal |
| Example 5 | Crospovidone | ProSolv SMCC 90 | Dry |
| Example 6 | Crospovidone | ProSolv SMCC 90 | Normal |
| Example 7 | Croscarmellose sodium | ProSolv SMCC 90 | Normal |
| Example 8 | Croscarmellose sodium | ProSolv SMCC 90 | Dry |
| Example 9 | Crospovidone | Avicel PH 102 | Half dry, half normal |
| Example 10 | Crospovidone | Avicel PH 102 | Half dry, half normal |

In Table 12, the normal (*) excipients mean that they were exposed to 40 % RH for 24 hours at ambient temperature; and the dry (#) excipients mean that they were dried for 4 hours at ambient temperature in a vacuum chamber. As a result of drying, the water activity of each excipient was reduced as shown in Table 13.

| **Table 13** | | | | |
|---|---|---|---|---|
| | Water activity of excipients in Samples | | | |
| | Water activity normal (*) | Temperature (°C) | Water activity dry (#) | Temperature (°C) |
| Crospovidone | 20.6 | 22.8 | 9.5 | 21.1 |
| Croscarmellose sodium | 16.4 | 20.7 | 4.4 | 21.9 |
| Hydroxypropyl Cellulose, Low substituted | 23.5 | 22.9 | 8.5 | 21.6 |
| Microcrystalline Cellulose | 32.7 | 21.9 | 14.9 | 21.0 |
| Silicified Microcrystalline Cellulose | 35.9 | 21.7 | 15.2 | 20.9 |
| Lactose anhydrous | 28.6 | 22.8 | 14.8 | 22.5 |
| Mg stearate | 26.6 | 22.8 | 15.9 | 21.1 |

### Influence of water activity of the formulation on physical stability of Canagliflozin

The influence of water activity of the formulation on physical stability of Canagliflozin was further tested. Prior storage at stress stability conditions samples were exposed to different relative humidity (30, 40, 50 and 60 RH %). After storage at elevated temperature (50°C) for 14 days samples were tested for water activity and polymorphic form. It was established that Canagliflozin was physically stable when water activity was ≤ 0.4. Results confirmed that appropriate dryness of the samples is critical for product stability (see Table 14, showing Examples 2 and 7 as exemplary samples).

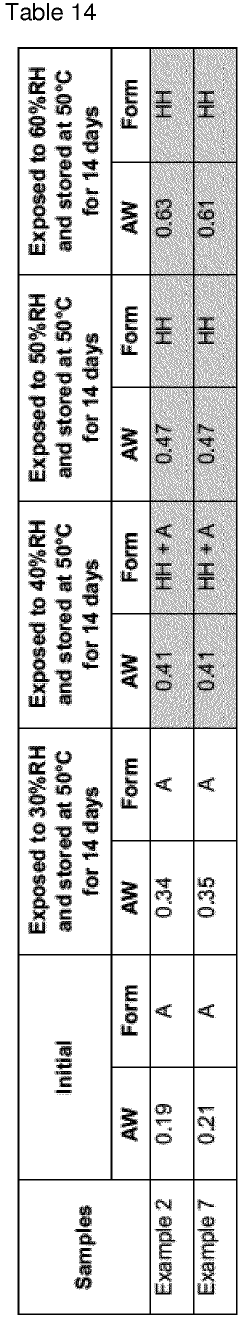

Further, 6 samples were prepared according to the composition as shown in Table 15. The samples were prepared in accordance with Examples 1 to 10 as above. The amorphous Canagliflozin was micronized in Examples 11 and 13, but not micronized in Examples 12 and 14. In Examples 15 and 16, half of the amorphous Canagliflozin was micronized.

**Table 15**

| | **Canaglifliozin (mg)** | **Croscarmellose sodium (mg)** | **L HPC LH11 (mg)** | **Mg stearate (mg)** | **ProSolv SMCC 90 (mg)** | **Sodium Lauryl Sulfate (mg)** | **Poloxamer (mg)** | **Blanose LF (Na CMC) (mg)** | **Total (mg)** |
|---|---|---|---|---|---|---|---|---|---|
| **Example 11** | 100 | 10 | 10 | 2 | 56 | 5 | - | 17 | 200 |
| **Example 12** | 100 | 10 | 10 | 2 | 73 | 5 | - | - | 200 |
| **Example 13** | 100 | 10 | 10 | 2 | 73 | - | 5 | - | 200 |
| **Example 14** | 100 | 10 | 10 | 2 | 56 | - | 5 | 17 | 200 |
| **Example 15** | 50+50 | 10 | 10 | 2 | 67 | 2.5 | - | 8.5 | 200 |
| **Example 16** | 50+50 | 10 | 10 | 2 | 67 | 2.5 | - | 6.5 | 200 |

The influence of water activity on physical stability of Canagliflozin were further tested for Examples 11, 12, 15 and 16. The results are shown in Table 16 which confirms that appropriate dryness of the samples is critical for product stability.

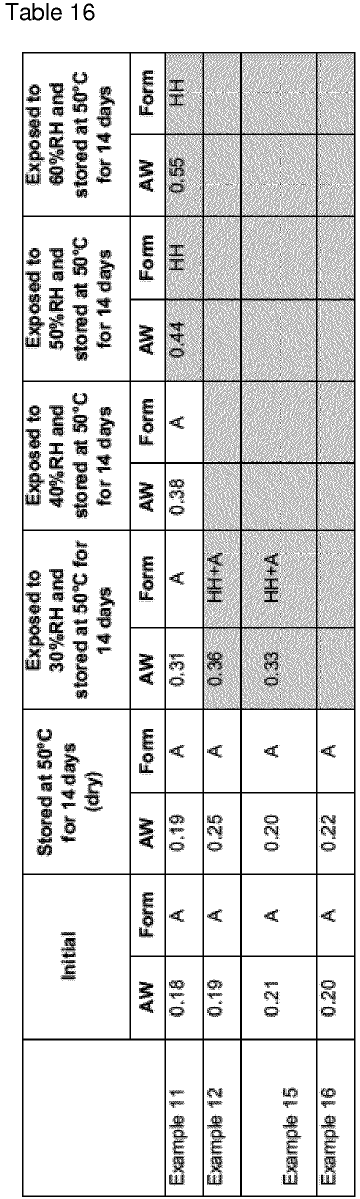

### EXAMPLE C - COATING

### Coating Examples

### Coating mixture 1

| | Proportion (%) |
|---|---|
| Kollicoat^{®} Protect | 12 |
| Talc | 5 |
| Titanium dioxide | 3 |
| Water | 80 |

Qualitative composition of Kollicoat^{®} Protect:

| | |
|---|---|
| Polyvinyl alcohol-polyethylene glycol graft copolymer | 55-65 % |
| Polyvinyl alcohol | 35-45 % |
| Silicon dioxide | 0.1-0.3 % |

Kollicoat^{®} Protect, talc and pigments are dissolved or dispersed in water. The obtained coating mixture is applied onto the tablets prepared according to the present invention by using spray-coating in perforated drums. Film coating represents 2.5 % w/w of tablet core.

### Coating mixture 2

| | Proportion (%) |
|---|---|
| Opadry II (PVA based) | 20 |
| Water | 80 |

Qualitative composition of Opadry II (PVA based):
Polyvinyl alcohol-partially hydrolyzed, Titanium dioxide, Talc, Macrogol.

Opadry II is dissolved or dispersed in water. The obtained coating mixture is applied onto the tablets prepared according to the present invention by using spray-coating in perforated drums. Film coating represents 4.5 % w/w of tablet core.

### Coating mixture 3

| | Proportion (%) |
|---|---|
| Hydroxypropylmethyl cellulose 603 | 4.82 |
| Hydroxypropyl cellulose EF | 0.54 |
| Macrogol | 0.8 |
| Talc | 0.4 |
| Titanium dioxide | 1.44 |
| Water | 9.2 |
| Ethanol | 82.8 |

Excipients are dissolved or dispersed in water/ethanol. The obtained coating mixture is applied onto the tablets prepared according to the present invention by using spray-coating in perforated drums. Film coating represents 3 % w/w of tablet core.

### Coating mixture 4

| | Proportion (%) |
|---|---|
| Hydroxypropyl cellulose EF | 6.38 |
| Stearic acid | 1.12 |
| Triethyl citrate | 0.5 |
| Talc | 1 |
| Titanium dioxide | 1 |
| Ethanol absolute | 90 |

Excipients are dissolved or dispersed in absolute ethanol. The obtained coating mixture is applied onto the tablets prepared according to the present invention by using spray-coating in perforated drums. Film coating represents 2.5 % w/w of tablet core.

## Claims

1. A formulation comprising a non-stoichiometric hydrate of Canagliflozin or an amorphous Canagliflozin, and at least one of excipients, wherein the formulation is defined by a water activity of ≤ 0.3, determined at room temperature.

2. The formulation of claim 1, wherein the non-stoichiometric hydrate of Canagliflozin is HxA, HxB or a mixture of HxA and HxB, preferably is HxA.

3. The formulation of claim 1 or 2, wherein HxA is the only detectable crystalline form of Canagliflozin.

4. The formulation of any one of preceding claims, wherein said at least one of excipients includes at least one type of excipient whose water activity was reduced by at least 30%.

5. The formulation according to any of the preceding items, wherein the water activity is determined with a reference humidity measuring instrument, preferably with the measuring instrument testo 950.

6. The formulation of any one of preceding claims, wherein the at least one of excipients are selected from:
fillers selected from the group consisting of microcrystalline cellulose, silicified microcrystalline cellulose, lactose monohydrate; preferably selected from microcrystalline cellulose and/or silicified microcrystalline cellulose;
disintegrants selected from the group consisting of croscarmellose sodium, crospovidone, low-substituted hydroxypropyl cellulose (L-HPC), preferably selected from croscarmellose sodium;
solubilizers selected from the group consisting of surfactants and solubilizing polymers, preferably selected from the group consisting of sodium lauryl sulfate and
hydroxypropylmethyl cellulose (HPMC), more preferably are sodium lauryl sulfate or a mixture of sodium lauryl sulfate and HPMC; and
lubricants selected from magnesium stearate.

7. The formulation of claim 6, comprising as excipients sodium lauryl sulfate or a mixture of sodium lauryl sulfate and HPMC, preferably wherein the concentration of sodium lauryl sulfate or each of sodium lauryl sulfate and HPMC in the mixture is more than 0.1% (w/v), preferably 0.5 to 2.0% (w/v), more preferably 0.8 to 1.2% (w/v), most preferably 1.0% (w/v).

8. A pharmaceutical composition, comprising a dry formulation according to any one of claims 1 to 7, wherein the composition is formulated in a single dosage form, preferably a tablet, more preferably a film-coated tablet.

9. The pharmaceutical composition of claim 8, which is incorporated into a moisture protective packaging, preferably wherein the moisture protective packaging is selected from the group consisting of aluminium blister, aclar blister, glass bottle containing desiccant and HDPE bottle containing desiccant.

10. The formulation of any of claims 1 to 7 or the pharmaceutical composition of claim 8 or 9, obtained by a dry manufacturing process, preferably wherein the dry manufacturing process is a direct compression, a dry granulation such as a roller compaction and a slugging, and a moisture activated dry granulation, more preferably is a direct compression or a dry granulation.

11. The formulation of any of claims 1 to 7 or the pharmaceutical composition of claim 8 or 9, obtained under dry atmosphere conditions (relative humidity (RH) of ≤ 30%) during processing.

12. A process for manufacturing a formulation comprising Canagliflozin, (i) including dry atmosphere conditions (relative humidity (RH) of ≤ 30%) during processing; and/or (ii) wherein the resulting formulation is defined by a water activity of ≤ 0.3.

13. The process of claim 12, including a dry manufacturing process, preferably wherein the dry manufacturing process is a direct compression, a dry granulation such as a roller compaction and a slugging, and a moisture activated dry granulation, more preferably is a direct compression or a dry granulation.

14. The process of claim 12 or 13, wherein Canagliflozin is in the form of a non-stoichiometric hydrate or in an amorphous form, preferably is HxA.

15. A container comprising a formulation according to any of claims 1 to 7, the pharmaceutical composition according to claim 8 or 9, the formulation according to claim 10 or 11, or the formulation obtainable by the process according to any of claims 12 to 14, and a means to keep the water activity of the formulation or composition at below 0.3, determined at room temperature.

16. A formulation according to any of claims 1 to 7 or a pharmaceutical composition according to claim 8 or 9 for use as a medicament, preferably in the treatment of diabetes mellitus, diabetic retinopathy, diabetic neuropathy, diabetic nephropathy, delayed wound healing, insulin resistance, hyperglycemia, hyperinsulinemia, elevated blood levels of fatty acids, elevated blood levels of glycerol, hyperlipidemia, obesity, hypertriglyceridemia, Syndrome X, diabetic complications, atherosclerosis and/or hypertension.

17. A pharmaceutical combination comprising a formulation according to any of claims 1 to 7 or a pharmaceutical composition according to claim 8 or 9, and metformin.
